# EUROPEAN PATENT APPLICATION

(11) **EP 4 789 727 A1**
(43) Date of publication of application: **12.08.2026**
(21) Application number: 25157230.1
(22) Date of filing: 11.02.2025
(51) Int. Cl.: A61P 37/08, C07K 16/18, C07K 16/28, C07K 16/42

(54) **ANTIBODY FOR USE IN A THERAPY OF AN IGE-ASSOCIATED DISEASE**

(71) Applicant: Universitätsklinikum Hamburg-Eppendorf, 20246 Hamburg (DE)
(72) Inventor: MÖLLER, Constantin, 20246 Hamburg (DE); FEHSE, Boris, 20246 Hamburg (DE); RIECKEN, Kristoffer, 20246 Hamburg (DE); EDEN, Thomas, 20246 Hamburg (DE); HAMBACH, Julia, 20246 Hamburg (DE); NOLTE, Friedrich, 20246 Hamburg (DE); SCHAFFRATH, Alessa Zoe, 20246 Hamburg (DE)
(74) Representative: Stüven, Ralf

(57) **Abstract**

The invention provides a multispecific antibody for use in a therapy of an IgE-associated disease, for example, an anti-allergy therapy, the antibody specifically targeting membrane-bound IgE (mIgE) on the surface of target cells, for example B cells, and being configured to engage immune effector cells for the elimination of the target cells. The antibody of the invention comprises
a) a first binding domain binding to the CεmX domain of the immunoglobulin E extracellular membrane-proximal domain, EMPD, the first binding domain comprising a complementarity-determining region, CDR, of the single-chain variable fragment antibody 2E3E10;
b) a second binding domain binding to an epitope on the surface of an immune effector cell.

## Description

The invention relates to an antibody for use in a therapy of IgE-associated disease, in particular for use in anti-allergy therapy.

IgE-associated diseases include allergic diseases, chronic urticaria, neoplasms, IgE multiple myelomas or non-Hodgkin lymphomas (see, e.g., González AA, IgE Diseases, 2021, Clin Res Trials, doi: 10.15761/CRT.1000333; Navinés-Ferrer A, Serrano-Candelas E, Molina-Molina GJ, Martin M., 2016, IgE-Related Chronic Diseases and Anti-IgE-Based Treatments. J Immunol Res. 2016:8163803, doi: 10.1155/2016/8163803; Macro, M., André, I., Comby, E., Chèze, S., Chapon, F., Ballet, J. J., Reman, O., Troussard, X., 1999, IgE Multiple Myeloma. Leukemia & Lymphoma, 32(5-6), 597-603, doi:10.3109/10428199909058419; Sundström C, Nilsson K, Brenning G., 1988, IgE-producing low-grade non-Hodgkin lymphoma. Eur J Haematol. 1988 Oct;41(4):388-95. doi: 10.1111/j.1600-0609.1988.tb00214.x). Among IgE-associated diseases allergic diseases are the most common. Allergic diseases are aberrant immune responses to otherwise innocuous molecules, known as allergens. Affecting approximately 30% of the world population, immunoglobulin-E (IgE-) associated allergic diseases, including allergic asthma, allergic rhinitis, allergic dermatitis, allergic conjunctivitis, as well as food and drug allergies (Balbino et al., 2018; Gupta et al., 2019; Ling et al., 2023; Wang et al., 2023) are the most prevalent types of allergic diseases with continuously rising incidence (Platts-Mills, 2015; Bergmann et al., 2016; Sampath et al., 2021; Warren et al., 2023).

The treatment of IgE-associated allergies has traditionally focused on avoidance of antigen contact, which is not always feasible. Following allergen exposure, traditional pharmacotherapy is employed to alleviate symptoms (Bousquet et al., 2020; Sampath et al., 2021; Wang et al., 2023). However, traditional pharmacotherapy, which primarily involves H1-antihistamines, corticosteroids and leukotriene receptor antagonists, requires continued, potentially lifelong administration. Moreover, these therapies are not free of adverse effects, and patients often experience insufficient symptom relief when using pharmacotherapy alone (Bernstein et al., 2016; Wang et al., 2023).

The only established therapy that can achieve long-term allergen tolerance is allergen immunotherapy (AIT). However, this potentially curative therapy is not available for all allergens, typically requires three years of treatment to achieve long-term efficacy and can lead to adverse reactions (Xiong et al., 2022; Durham & Shamji, 2023; Wang et al., 2023). Moreover, potential long-term side effects of AIT remain to be elucidated.

There have been many efforts to identify new therapeutic options for IgE-associated allergic diseases. IgE itself has been considered a promising therapeutic target for treating IgE-mediated allergic diseases, as it plays a central pathophysiological role (Jardieu, 1995; Hu et al., 2018). This has been underlined by the clinical benefits of anti-IgE monoclonal antibodies like Omalizumab, which have demonstrated that depletion of IgE can be a safe and effective therapeutic approach (Pelaia et al., 2018; Gasser et al., 2020; Okayama et al., 2020).

Typically, after loss of tolerance to an otherwise harmless allergen during an initial sensitization stage, allergen-specific IgE is produced. This allergen-specific IgE then binds to Fcε receptors on mast cells, basophils, or eosinophils (Akdis et al., 2023). Upon re-exposure, the allergen binds to the specific IgE, crosslinking the IgE-Fcε complexes and thus leading to the release of inflammatory mediators such as histamine and leukotrienes. This cascade results in early-phase allergic symptoms that can culminate in anaphylaxis (Bousquet et al., 2020; Wang et al., 2023).

Physiologically, IgE is involved mainly in immune responses against parasites and is also thought to play a protective role against venoms (Wu & Zarrin, 2014; He et al., 2015; Balbino et al., 2018). However, nowadays in industrial countries, allergies are the main cause of elevated IgE serum levels (Joshi et al., 2009), as parasitic infections have become less common and generally are well treatable. Therefore, depleting soluble IgE using Omalizumab is considered to be safe and well tolerated (Pelaia et al., 2018; Tharp et al., 2019; Wood et al., 2024). Whereas Omalizumab has demonstrated the principal feasibility of anti-IgE therapy, it is not curative but requires regular administration every 2 to 4 weeks (Hanania et al., 2022). A potentially curative therapy could work by permanently eliminating IgE production. However, this is complicated by the still uncertain mechanisms of IgE production and the incomplete characterization of IgE-producing cells (Eckl-Dorna et al., 2012; Davies et al., 2013; Eckl-Dorna & Niederberger, 2013; Wu & Scheerens, 2014; Eckl-Dorna et al., 2019; Xiong et al., 2022; Ling et al., 2023). The current understanding is that IgE is produced by IgE-producing plasma cells (PC), which can be long-lived plasma cells (LLPC) or short-lived plasma cells (SLPC), and themselves differentiate from IgE-class-switched B cells (Ling et al., 2023).

IgE-class-switched B cells are believed to express membrane IgE (mIgE) as part of their B cell receptor (BCR) complexes (Achatz & Lamers, 1997; Achatz et al., 2008). This mIgE is believed to be downregulated to an indefinite level upon differentiation into an antibody-secreting PC (Achatz et al., 2008).

In contrast to their soluble forms, membrane-bound immunoglobulins of all isotypes have three additional structural features: a cytoplasmic domain, a transmembrane domain, and an extracellular membrane-proximal domain (EMPD). As a result of alternative splicing, mIgE can exist in one of two functional forms of the ε_{EMPD} domain: either a 14 amino acid long form εₛₕₒᵣₜ or a 66 amino acid long form ε_{long}, with ε_{long} thought to be predominantly expressed (Zhang et al., 1992; Achatz et al., 2008). The EMPD, particularly the unique 52 amino acid long extra domain CεmX only present in ε_{long} (Chen et al., 2002; Wan et al., 2010), is a promising antigenic site to target IgE-expressing B cells (Chen et al. 2010; Wu et al., 2012). Several antibodies targeting this domain have been developed (Scheerens et al. 2012; Feichtner et al., 2008; Brightbill et al., 2010; Yueh-Hsuan Chan et al., 2014; Ma & Fay, 2017; Vigl et al., 2017; WO 2014/165028 A1; WO 2019/085902 A1; US 8460664 B2).

One such antibody, Quilizumab, which targets the CεmX-containing domain of mIgE, (Brightbill et al., 2010; Brightbill et al., 2014; Gauvreau et al., 2014) has been clinically explored up to a phase-II trial for the treatment of allergic asthma. Although Quilizumab was well tolerated, considered safe to be used and able to reduce total as well as allergen-specific sIgE levels, it showed no clinical benefit in a large cohort of patients with allergic-asthma and was therefore discontinued (Gauvreau et al., 2014; Harris et al., 2016).

One potential way to improve the efficacy of mIgE-targeting therapies is to use alternative platforms besides traditional antibodies, such as immune cell engagers (ICEs) like, for example, bispecific engagers. Bispecific engagers are a type of bispecific antibody (BsAb) and a promising class of immunotherapeutic agents that have gained increasing attention in recent years (Goebeler & Bargou, 2020; Klein et al., 2024). These molecules have been designed to simultaneously bind to immune effector cells, such as T cells or natural killer (NK) cells, and to target cells of interest bringing them into close proximity. Thus, the immune effectors are not only "engaged" but activated to selectively target and eliminate the respective target cell, e.g. cancer cells.

The most prominent bispecific engager is Blinatumomab, an anti-CD3ε - anti-CD19 bispecific T cell engager (BiTE), approved for the treatment of adult and pediatric patients with CD19-positive B-cell precursor acute lymphoblastic leukemia (Burt et al., 2019). At least two separate research groups have created several analogous BiTE constructs that target mIgE (Kirak & Riethmüller, 2015; Rodak et al., 2021). Most of those constructs bind both mIgE and sIgE, which is likely to significantly reduce the efficacy of the mIgE-targeting treatment. In contrast, Rodak and colleagues developed a CεmX-targeting engager based on Quilizumab that does not bind to sIgE. However, this engager showed only low levels of staining compared to the other constructs and was unable to activate T cells and therefore ineffective (Rodak et al., 2021). Nevertheless, the authors suggested that a similar construct that does not interact with sIgE but elicits potent T cell activation could be advantageous for the specific killing of IgE-producing cells (Rodak et al., 2021). Vogel (2022) describes an IgE-CεmX-BiTE antibody construct combining the anti-EMPD scFv of the antibody 47H4 (Brightbill et al. 2010) and a CD3-specific scFv.

Despite the diverse approaches and recent progress in the treatment of allergic diseases there is still a need for novel agents for use in anti-allergy therapy, in particular agents for use in the treatment of IgE-mediated diseases. It is an object of the invention to provide such an agent.

The object is solved, in a first aspect, by an antibody for use in an anti-allergy therapy, the antibody comprising
a) a first binding domain binding to the CεmX domain of the immunoglobulin E extracellular membrane-proximal domain, EMPD, the first binding domain comprising a complementarity-determining region, CDR, of the single-chain variable fragment antibody 2E3E10;
b) a second binding domain binding to an epitope on the surface of an immune effector cell.

The invention provides a novel agent for use in a therapy against IgE-associated diseases, in particular IgE-mediated allergic diseases. The agent of the invention is a multispecific antibody, for example a bispecific antibody, targeting cells, e.g. B cells, expressing membrane-bound IgE (mIgE). The antibody is configured to bind to an epitope on a surface protein (e.g., CD3 or CD 16) present on the surface of an immune effector cell, for example, a T-cell or natural killer cell, and to the CεmX domain of mIgE present on the surface of the target cell, and to engage the immune effector cell to elicit the immune-cell-mediated killing of the target cell. The therapy thus involves the cytotoxic elimination of an mIgE-expressing target cell by engaging the target cell with an immune effector cell, e.g. a T-cell or a natural killer (NK) cell. The antibody of the invention can advantageously be used for the specific killing of IgE-producing cells, and could thus provide a causal treatment of, for example, allergic diseases by eliminating IgE-producing cells (plasma cells, plasmablasts) and IgE-positive B and memory B cells. In addition, the antibody of the invention can also be used for the specific killing of IgE-expressing cancer cells.

The antibody of the invention is an artificial antigen-binding construct comprising at least two binding domains fused together, the binding domains being arranged in a manner enabling the antibody to engage an mIgE-expressing target cell with an immune effector cell by simultaneously binding on the one hand to mIgE on the surface of the target cell and on the other hand to an epitope on the surface of the immune effector cell.

The term "simultaneously binding" does not exclude that the antibody can bind to both epitopes one after another, but means that both epitopes are bound by the antibody over a common period of time. The antibody can be an antibody composed of a single polypeptide or of two or more polypeptides being covalently bound together, e.g., via disulfide bonds, or forming a peptide complex (dimer or oligomer) composed of separate polypeptide chains (subunits) held together by noncovalent forces to form a quaternary structure.

The term "immunological synapse" is used herein in relation to a cell-cell junction with a synaptic cleft stabilized by an adhesion molecule, e.g., a bispecific antibody, enabling cell-cell communication between an immune cell and a target cell. The term "cytolytic synapse" relates to an immunological synapse formed for cytolytically eliminating a target cell by or with the aid of an immune cell.

The term "multispecific antibody", abbreviated MsAb, relates to an artificial antibody with more than one specificity, i.e. an antibody that can simultaneously bind two or more separate and unique epitopes, the epitopes being on separate antigens or on the same antigen. As used herein, the term also encompasses an artificial antibody that can simultaneously bind to the same epitope or different epitopes on two or more separate but identical antigens, e.g., two or more separate surface molecules of the same kind (e.g., CD3) present on, e.g., two separate cells. The term also encompasses bispecific antibodies. An example of a multispecific antibody is an antibody that can bind simultaneously to two or more target proteins and/or bridge two different cells.

The term "bispecific antibody", abbreviated BsAb, relates to an artificial antibody that can simultaneously bind two separate and unique epitopes, the epitopes being on two separate antigens or on the same antigen (see, for example, the references above). As used herein, the term also encompasses an artificial antibody that can simultaneously bind to the same epitope or different epitopes on two separate but identical antigens, e.g., two separate surface molecules of the same kind (e.g., CD3) present on the same or on two separate cells. As used herein, the term "bispecific antibody" should not be construed as meaning that further specificities, for example a third specificity, are excluded. The term may also be used here in connection with an antibody having a third binding specificity, for example, a binding specificity against albumin. Bispecific antibodies are configured to have two binding domains (paratopes) with preferably different defined specificities. They can be produced, for example, by various chemical, biochemical or biotechnological means, and exist in many different formats (see, e.g., Brinkmann & Kontermann 2017; Elshiaty et al. 2021; Voigt et al. 2022). BsAbs may or may not include a functional antibody Fc-region.

The term "complementarity-determining region", CDR, relates to polypeptide segments of the variable chains of an antibody or T-cell receptor (TCR), with which the antibody or T-cell receptor binds to its specific antigen, and which structure/sequence determines the binding activity of the respective antibody or TCR. Each variable domain of an antibody or TCR has usually three non-consecutively arranged CDRs (CDR1, CDR2 and CDR3). The CDRs of the light chain are designated LCDR1, LCDR2, and LCDR3, the CDRs of the heavy chain HCDR1, HCDR2, and HCDR3.

The term "comprising a complementarity-determining region, CDR, of the single-chain variable fragment antibody 2E3E10" in relation to the first binding domain refers to any of the CDRs of scFv 2E3E10. The term encompasses not only a CDR having or comprising any of the amino acid sequences QSLLYSSNQKNY, WAS, QQYYSYPWT, GYSITSGYY, ISYDGSN, ARDYGGFDY (SEQ IDs 2-6), but also a CDR having or comprising an amino acid sequence with not more than 45%, preferably not more than 40%, 35%, 30%, or 25% amino acid replacements, or with not more than one (in particular in WAS), two or three amino acid replacements that do not, however, essentially effect binding activity of the CDR.

The term "epitope" (also called "antigenic determinant") relates to a portion of a substance, e.g., a molecule, molecular structure, or particulate matter, called antigen, to which a specific antibody binds with its binding domain. The term "antigen" relates to a substance, e.g., a molecule, molecular structure, or particulate matter, that comprises at least one epitope and that is capable of stimulating an immune response, in particular is recognized by an antibody. The terms "antigen" and "epitope" may be used synonymously here, in particular in a case where two epitopes are present on different molecules. For example, saying that the bispecific antibody binds an epitope on CD3 is equivalent to saying that the BsAb binds to the CD3 antigen, if not clearly stated otherwise.

The term "therapy" relates to a medical treatment of a disease or disorder. The term "immunotherapy" relates to a medical treatment of a disease or disorder by activating or suppressing the immune system of the subject to be treated. An immunotherapy can, for example, be a cell-based therapy, i.e., a therapy involving the activation or suppression of immune effector cells of a subject. The terms "allergy therapy" or "allergy immunotherapy" relates to an immunotherapy, in particular an antibody and/or cell-based immunotherapy, for treating allergy.

The terms "IgE-associated disease", "IgE-related disease" or "IgE disease" refer to a disease or disorder that is characterized by, involves or results from an imbalance or abnormal production, expression or activity of immunoglobulin E. Examples of IgE-associated diseases comprise, but are not limited to, for example, allergic diseases, chronic urticaria (CU), neoplasms, IgE multiple myelomas or non-Hodgkin lymphomas (see, e.g., González AA, IgE Diseases, 2021, Clin Res Trials, doi: 10.15761/CRT.1000333; Navinés-Ferrer A, Serrano-Candelas E, Molina-Molina GJ, Martin M., 2016, IgE-Related Chronic Diseases and Anti-IgE-Based Treatments. J Immunol Res. 2016:8163803, doi: 10.1155/2016/8163803; Macro, M., André, I., Comby, E., Chèze, S., Chapon, F., Ballet, J. J., Reman, O., Troussard, X., 1999, IgE Multiple Myeloma. Leukemia & Lymphoma, 32(5-6), 597-603, doi:10.3109/10428199909058419; Sundström C, Nilsson K, Brenning G., 1988, IgE-producing low-grade non-Hodgkin lymphoma. Eur J Haematol. 1988 Oct;41(4):388-95, doi: 10.1111/j .1600-0609.1988.tb00214.x).

The term "allergy" or "allergic disease" refers to a condition or disease caused by hypersensitivity of the immune system to an ordinarily harmless substance, i.e. a condition or disease in which a person's immune system abnormally reacts to a foreign substance (allergen, antigen) in the environment that gets inside the person's body but is harmless to most people. Examples of allergens are pollens, foods, bee venom, pet dander, and house dust mite. The terms "IgE-mediated allergy", "IgE-associated allergy" or "IgE-related allergy" relate to a type I (immediate-type) hypersensitivity reaction provoked by immunological mechanisms involving the stimulation of the production of allergen-specific IgE antibodies by plasma cells in a subject upon exposure to the allergen, for example, a food allergen. Examples of IgE-mediated allergies are acute rhinitis, conjunctivitis, acute asthma, acute urticaria, angioedema and IgE-mediated anaphylaxis.

The term "involving the cytotoxic elimination of a target cell by engaging the target cell with an immune effector cell" in relation to a therapy relates to a therapy in which immune effector cells are brought in intimate contact with a target cell, e.g., using artificial molecules like bispecific antibodies, thus forming a cytolytic synapse leading to cell death of the target cell.

The terms "immune effector cell", IEC, "effector immune cell" or "effector cell" relate to cells of the immune system of the human body, i.e., cells that are capable of modulating, effecting or enhancing an immune response in response to stimulation. Examples of immune effector cells include natural killer (NK) cells, cytotoxic T lymphocytes (cytotoxic T cells, CD8+ T cells), B lymphocytes (B cells), helper T cells (T helper cells, CD4+ T cells), macrophages and dendritic cells. The term also encompasses genetically modified immune effector cells.

The term "cytotoxic elimination" relates to the killing of cells by immune effector cells.

The term "CεmX domain", also abbreviated CεmX or "M1'", is a 52 amino acid long section of the predominant long human isoform (ε_{long}) of the extracellular membrane-proximal domain (EMPD) of membrane-bound immunoglobulin E (mIgE). CεmX is not expressed in secreted IgE (sIgE) The sequence (with two allelic forms differing by a leucine or valine at position 16, Chu et al. 2014; SEQ ID NO: 1) is as follows;
GLAGGSAQSQRAPDR[V/L]LCHSGQQQGLPRAAGGSVPHPRCHCGAGRADWPGPP

The term "extracellular membrane-proximal domain", abbreviated EMPD, relates to a section of membrane-bound immunoglobulin E (mIgE) arranged between the most C-terminal constant Ig domain (CH4) and the transmembrane domain. The EMPD exists in two forms, a long form comprising CεmX, and a short form lacking CεmX. The long form consists of 67 amino acids (52 amino acids of CεmX plus 15 additional C-terminal amino acids). The term "extracellular membrane-proximal domain", EMPD, relates in particular to the long form comprising CεmX.

The term "single-chain variable fragment antibody 2E3E10", short "scFv 2E3E10", "scFv antibody 2E3E10" or "2E3E10", relates to a monoclonal antibody designated "2E3E10" (see, e.g., US 2018/0022826 A1) specific to CεmX, , in particular the N-terminal portion of CεmX). The CDRs of scFv 2E3E10 are as follows (LCDR = light chain CDR, HCDR = heavy chain CDR):

| CDR | Sequence | SEQ ID NO: |
|---|---|---|
| LCDR1 | QSLLYSSNQKNY | 2 |
| LCDR2 | WAS | - |
| LCDR3 | QQYYSYPWT | 3 |
| HCDR1 | GYSITSGYY | 4 |
| HCDR2 | ISYDGSN | 5 |
| HCDR3 | ARDYGGFDY | 6 |

The term "target cell" relates to a living cell, e.g., a tumor cell or a B cell, being a target for the binding of the antibody, and/or for cytotoxic elimination.

The terms "mIgE-positive cell", "mIgE+ cell" or "mIgE-expressing cell" relates to cells carrying, on their cell surface, the long variant of membrane-bound immunoglobulin E (IgE), i.e. the IgE variant comprising EMPD. An example of such cells are IgE-class-switched B cells.

The term "T cell engager" (TCE) relates to a binding molecule, e.g. a protein, simultaneously binding a target antigen on a target cell, e.g., an IgE class-switched B cell, and an antigen on a T cell to form an artificial immune synapse that is independent of the T cell receptor (TCR) (see, for example, Vafa & Trinklein (2020)). A bispecific antibody can, for example, be a T cell engager (bispecific T-cell engager, BiTE) and circumvent HLA restriction. In case of a bispecific T cell engager, the term bispecific T-cell engager, abbreviated, "BTCE" or "bTCE", may, for example, be used. Similarly, the term "NK cell engager" (NKCE) relates to a protein simultaneously binding a target antigen on a target cell, e.g., a tumor cell or B cell, and an antigen on an NK cell to form an artificial immune synapse. Again, a bispecific antibody can function as an NK cell engager (BiKE, BiNKE or BNKCE) (see, e.g., Demaria et al. (2021); Sivori et al. (2019); Felices et al. (2016)).

The terms "BiTE" or "BiKE" may be used herein as short forms not only referring to a bispecific antibody but also to an antibody comprising a third or further binding domains.

The terms "anti-CεmX BiTE" and "anti-CεmX BiKE" may be used here in relation to multispecific (in particular bispecific) antibodies directed against the CεmX domain of membrane-bound IgE.

The terms "HLE-BiTE" or "HLE-BiKE" may be used herein as short forms referring to half-life extended variants of a multispecific antibody of the invention.

The term "blinatumomab" refers to the bispecific T-cell engager "Blinatumomab" (CAS No. 853426-35-4), which is also sold under the name "Blincyto". The antibody is composed of two single-chain antibody (scFv) domains, a CD3 binding domain for targeting T-cells and a CD19 binding domain for targeting B cells linked by a 5 amino acids linker (see, e.g., Burt et al. 2019). The term "blinatumomab anti-CD3 scFv antibody" refers to the anti-CD3 binding domain (scFv) L2K (L2K-07; see Mocquot et al. 2022) of Blinatumomab.

The term "nanobody" refers to a single-domain antibody (sdAb), i.e. an antibody composed of the recombinant variable domain of a heavy-chain-only antibody (see, e.g., Jin et al. 2023). Nanobodies are antibody fragments derived from heavy-chain only IgG antibodies found in the Camelidae family. The terms "VHH antibody" (variable heavy domain of heavy chain) or "heavy chain-only antibody" may also synonymously be used.

The term "single-domain anti-CD3 antibody CD3 3D-H11" refers to a single-domain antibody binding to CD3 (see, Moradi-Kalbolandi et al. 2020, WO 2010037838 A2).

The term "single domain anti-CD16 antibody c21" refers to a single-domain antibody binding to CD16 (s. Behar et al. 2008; Hambach et al. 2022; GenBank ABQ52435.1)

The term "anti-HSA antibody Alb11" refers to an antibody binding to human serum albumin (HSA) designated "Alb11" or "Alb-11" (see, e.g., WO 2012/8175400 A1).

The terms "CD3" (cluster of differentiation 3) or "CD3 antigen", also "CD3 receptor", have their common meanings and relate to a protein complex comprising four different transmembrane proteins, in mammals a gamma chain, a delta chain and two epsilon chains. The protein complex associates with the T-cell receptor (TCR). CD3 is, almost exclusively, present on mature T cells. The term "CD3ε" relates to the epsilon chain of CD3.

The terms "CD16" (cluster of differentiation 16) or "CD16 antigen", also known as FcγRIII, is a membrane receptor protein. The term encompasses the two variants existing in humans, CD16a (FcγRIIIa) and CD16b (FcγRIIIb). CD16 is found on the surface of natural killer cells, neutrophils, monocytes, macrophages, and certain T cells.

Terms in the format of "first surface protein x second surface protein", e.g., "mIgExCD3", "mIgExCD16", "CεmXxCD3", or "CεmXxCD16", relate to antibodies, e.g. bispecific antibodies, having a first paratope binding to an epitope on the first surface protein (e.g. mIgE) and a second paratope binding to an epitope on a second surface protein (e.g. CD3). An "mIgExCD3" antibody is thus an antibody, e.g., bispecific antibody, binding, with a first binding domain, to an epitope on mIgE and, with a second binding domain, to an epitope on CD3. As another example, a "CεmXxCD16" antibody is an antibody, for example, bispecific antibody, binding, with a first binding domain, to the CεmX domain of mIgE and, with a second binding domain, to an epitope on a CD16 protein. The CεmX domain and the CD16 protein may be on different cells.

The term "surface protein" relates to a membrane protein, i.e., a protein being part of or interacting with a biological membrane, in particular to a membrane protein having at least an extracellular portion. Surface proteins may be designated with their common abbreviations known to the skilled person, e.g. "mIgE", "CD3" or "CD16". A term like "CD#", "CD" standing for "Cluster of Differentiation" and "#" representing a number or a combination of a number and a letter (e.g., 3 or 45 or 1a), for example "CD45", is to be understood as also encompassing all isoforms, in case of CD45, for example, the isoforms CD45RA and CD45RO. Further, use of a term like "CD#", e.g., CD43, encompasses proteins being, for example, post-translationally modified, e.g., glycosylated or sialylated.

The term "present on the surface of a cell" in relation to a surface protein relates to the presence of the protein or a portion thereof on or the accessibility of the protein or a portion thereof from the extracellular side of a biological membrane. The term "expressed" in relation to a surface protein means that the protein is presented on the surface of the cell.

The term "half-life extended" relates to a compound, in the context of the present invention in particular to a protein, e.g. an antibody, being chemically and/or biochemically modified in a manner that the resulting modified compound has a longer serum half-life than the unmodified compound. Several means and techniques for half-life extension are known to the skilled person (see, e.g. Kontermann 2009, Seifert & Kontermann 2022). Proteins, e.g. antibodies, can, for example, be half-life extended by the addition of an anti-HSA (human serum albumin) antibody domain.

The term "pharmaceutically acceptable" means any substantially non-toxic material for mammals, especially humans, which does not substantially affect the effectiveness of the biological activity of the active ingredient. A "non-toxic" material is a material that is not toxic in the amount or concentration used per se or in the context of the composition administered. Such materials may include pharmaceutically acceptable concentrations of salts, buffers, preservatives, or the like. Non-limiting examples of pharmaceutically acceptable carriers include magnesium carbonate, magnesium stearate, talc, sugar, lactose, ethanol, glycerol, water, buffer solutions. The pharmaceutical composition may also comprise adjuvants and/or diluents.

The terms "having" or "comprising" in relation to an element or elements, e.g. a feature, component or sequence, are to be understood to not preclude other elements, unless expressly stated otherwise or the context does not clearly indicate otherwise. The term "having or comprising" in relation to an element or elements, e.g., a feature, a component, a sequence, or the like, means that the subject-matter characterized in this manner may either consist only of these elements, or may comprise other elements as well besides the element(s). A subject-matter defined in this manner thus encompasses a subject-matter which is limited to the elements mentioned, and a subject-matter which is not limited to the elements mentioned. For example, an antibody "having or comprising" an amino acid sequence A with 120 amino acids can be considered as either consisting only of these and no more than these 120 amino acids, or, alternatively, as consisting of these 120 and, optionally, other amino acids.

The term "secretory signal peptide" refers to a sequence motif targeting proteins for translocation across the endoplasmic reticulum membrane. For example, a signal peptide can be a 5-30 amino acid peptide present at the N-terminus of a secretory protein. The term "leader peptide" may synonymously be used for the term "signal peptide".

The term "separated by a linker" in relation to, for examples, two binding domains means that the binding domains do not follow one another directly without intervening amino acids, but are connected and separated from one another by a connecting sequence, a linker sequence. The term "GS linker" refers to a linker composed of glycine (G) and serine (S). The term "Whitlow/218 linker" refers to a linker with the originally published sequence GSTSGSGKPGSGEGSTKG (SEQ ID NO: 29) (Whitlow M, Bell BA, Feng SL, Filpula D, Hardman KD, Hubert SL, Rollence ML, Wood JF, Schott ME, Milenic DE, et al. An improved linker for single-chain Fv with reduced aggregation and enhanced proteolytic stability. Protein Eng. 1993 Nov;6(8):989-95. doi: 10.1093/protein/6.8.989) or a shortened variant thereof (GSTSGSGKPGSGEGS, SEQ ID NO: 30).

The invention provides, in a first aspect, an antibody for use in an anti-allergy therapy, the antibody being configured to bind, on the one hand, selectively to membrane-bound immunoglobulin E, mIgE, present on a target cell, e.g. a B cell, but not to secreted IgE, sIgE, and, on the other hand, simultaneously to an epitope on the surface of an immune effector cell, for example a CD3 or CD16 surface protein present on the surface of an immune effector cell, in order to enable the formation of a synapse between the target cell and the immune effector cell that would finally lead to the cell death of the target cell. The antibody construct comprises at least two paratopes, a first paratope binding to an epitope on the CεmX domain of the long isoform of mIgE, and a second paratope binding to a surface protein molecule on the surface of an immune effector cell, e.g., T cell or natural killer (NK) cell. For example, the antibody of the invention can be in the form of a bispecific T-cell engager, BiTE, or a natural killer cell engager, BiKE. In the former case, the antibody preferably comprises, besides the CεmX binding domain, a CD3 (e.g. CD3ε) binding domain, in the latter case preferably a CD16 (Fcγ receptor III) binding domain. The first or CεmX binding domain may synonymously also be designated as "2E3E10 domain".

In preferred embodiments, the antibody construct may further be configured to have an extended plasma half-life, e.g. by equipping the antibody with a third domain binding to human serum albumin (HSA). The antibody of the invention can be particularly advantageous in a therapy against IgE-mediated allergy, the therapy involving the cytotoxic elimination of IgE-producing target cells, e.g. plasma cells (PCs), by engaging the target cells with immune effector cells by means of the antibody of the invention.

The antibody of the invention comprises a first binding domain binding to the CεmX domain of the immunoglobulin E extracellular membrane-proximal domain, EMPD, the first binding domain comprising a complementarity-determining region, CDR, of the single-chain variable fragment antibody 2E3E10, for example at least one of the CDRs with sequences QSLLYSSNQKNY (SEQ ID NO: 2), WAS, QQYYSYPWT (SEQ ID NO: 3), GYSITSGYY (SEQ ID NO: 4), ISYDGSN (SEQ ID NO: 5), and ARDYGGFDY (SEQ ID NO: 6). Preferably, the first binding domain comprises at least two, at least three, at least four, at least five or six of the CDRs of the single-chain variable fragment antibody 2E3E10, more preferably at least one, two, three, four, five or all six of the CDRs with sequences QSLLYSSNQKNY (SEQ ID NO: 2), WAS, QQYYSYPWT (SEQ ID NO: 3), GYSITSGYY (SEQ ID NO: 4), ISYDGSN (SEQ ID NO: 5), and ARDYGGFDY (SEQ ID NO: 6).

In a preferred embodiment of the antibody for use in a therapy of an IgE-associated disease of the invention, the second binding domain is a binding domain binding to an epitope on CD3 or CD16. Other suitable epitopes on the surface of an immune effector cell, in particular on a protein on the surface of an immune effector cell, e.g., an epitope on NKG2D (CD314) or NKp30 (CD337), can, however, also be targeted.

In a preferred embodiment the antibody of the invention has an amino acid sequence comprising at least 200 consecutive amino acids, preferably at least 210 consecutive amino acids, more preferably at least 220, 230 or 240 consecutive amino acids, particularly preferred the full length, i.e. all consecutive amino acids of the sequence of scFv antibody 2E3E10 according to SEQ ID NO: 7, with the proviso that the antibody in any case comprises a CDR of scFv antibody 2E3E10, preferably two, three, four, five or all of the CDRs of scFv antibody 2E3E10.

The 2E3E10 domain preferably has or comprises the following amino acid sequence (SEQ ID NO: 7; CDRs underlined):

DIVMSQSPSSLAVSVGEKVTMSCKSSQSLLYSSNQKNYLAWYQQKPGQSPKLLIYWA
STRESGVPDRFTGSGSGTDFTLTISSVKAEDLAVYYCQQYYSYPWTFGGGTKLEIKRGS
TSGSGKPGSGEGSDVQLQESGPGLVKPSQSLSLTCSVTGYSITSGYYWNWIRQFPGNK
LEWMGSISYDGSNNYNPSLKNRISITRDTSKNQFFLKLNSVTTEDTATYYCARDYGGF
DYWGQGTTLTVSSAAA

In a preferred embodiment of the antibody of the invention the first binding domain comprising a complementarity-determining region, CDR, of the single-chain variable fragment antibody 2E3E10 is arranged further N-terminal than the second binding domain binding to an epitope on the surface of an immune effector cell, preferably to an epitope on CD3 or CD16. "Further N-terminal than" means an arrangement closer to the N-terminus. The first and second binding domain are thus, for example, arranged in the following order, in N-terminus to C-terminus direction: N-(2E3E10) - 2^{nd} domain-C. "(2E3E10)" stands for any binding domain comprising a CDR of scFv antibody 2E3E10, preferably two, three, four, five or six of the CDRs of scFv 2E3E10. The binding domains can be separated by a linker, e.g. a GS-linker, of suitable length, or not.

The antibody of the invention may comprise a suitable signal peptide, for example a human immunoglobulin leader peptide like the immunoglobulin heavy chain, IgH, signal peptide having or comprising the amino acid sequence MGWSCIILFLVATAT (SEQ ID NO: 23). Such a signal sequence allows for the secretion of the recombinant antibody of the invention. In an embodiment of the antibody of the invention, the signal peptide can be arranged at the N-terminal end of the protein, followed, separated or not by additional spacer amino acids, by the 2E3E10 domain.

In a preferred embodiment of the antibody of the invention, the second binding domain is a domain binding to CD3, e.g. the epsilon chain of CD3, CD3ε. Since such an embodiment of the invention is configured as a T cell engager, it may also be designated anti-CεmX BiTE.

In a preferred embodiment of an anti-CεmX BiTE of the invention, the second binding domain comprises at least one, preferably two, three, four or five of the CDRs of either the blinatumomab anti-CD3 scFv antibody or at least one, preferably two or three of the CDRs of the single-domain anti-CD3 antibody CD3 3D-H11. In case of the blinatumomab anti-CD3 scFv antibody the CDRs are preferably as follows:

| CDR | Sequence | SEQ ID NO: | |
|---|---|---|---|
| HCDR1 | RYTMH | | 8 |
| HCDR2 | YINPSRGYTNYNQKFKD | | 9 |
| HCDR3 | YYDDHYCLDY | | 10 |
| LCDR1 | RASSSVSYMN | | 11 |
| LCDR2 | DTSKVAS | | 12 |
| LCDR3 | QQWSSNPLT | | 13 |

In case of the single-domain anti-CD3 antibody CD3 3D-H11 the CDRs are preferably as follows:

| CDR | Sequence | SEQ ID NO: |
|---|---|---|
| CDR1 | DYGMS | 15 |
| CDR2 | DISWNGGSTYYADSVKG | 16 |
| CDR3 | MGEGGWGANDY | 17 |

In a preferred embodiment of an anti-CεmX BiTE of the invention, the second binding domain has or comprises one of the following sequences of SEQ ID NO: 14 or SEQ ID NO: 18:
SEQ ID NO 14 (Blinatumomab anti-CD3 domain, CDRs underlined): 
DIKLQQSGAELARPGASVKMSCKTSGYTFTRYTMHWVKQRPGQGLEWIGYINPSRGY
TNYNQKFKDKATLTTDKSSSTAYMQLSSLTSEDSAVYYCARYYDDHYCLDYWGQGT
TLTVSSVEGGSGGSGGSGGSGGVDDIQLTQSPAIMSASPGEKVTMTCRASSSVSYMNW
YQQKSGTSPKRWIYDTSKVASGVPYRFSGSGSGTSYSLTISSMEAEDAATYYCQQWSS
NPLTFGAGTKLELK
SEQ ID NO: 18 (single-domain anti-CD3 antibody "CD3 3D-H11", CDRs underlined):

EVQLVESGGGLVQPGGSLRLSCAASGFTFDDYGMSWVRQAPGKWLEWVSDISWNGG
STYYADSVKGRFTISRDNAENTLYLQMNSLKPDDTAVYYCAKMGEGGWGANDYWG
QGTQVTVSS

In another preferred embodiment of the antibody of the invention, the second binding domain is a domain binding to CD16, e.g. CD16a. Since such an embodiment of the invention is configured as a natural killer cell engager, it may also be designated anti-CεmX BiKE.

In a preferred embodiment of an anti-CεmX BiKE of the invention, the second binding domain comprises a complementarity-determining region, CDR, preferably at least two or three of the CDRs of the anti-CD16 single-domain antibody "c21" (see, for example Behar et al 2008; GenBank accession no. ABQ52435.1). Preferably, the CDRs are as follows

| CDR | Sequence | SEQ ID NO: |
|---|---|---|
| CDR1 | SGLTFSSY | 19 |
| CDR2 | REFVASITWSGRDTFYA | 20 |
| CDR3 | PWPVAAPRSGT | 21 |

In a preferred embodiment of an anti-CεmX BiKE of the invention, the second binding domain has or comprises the following sequence of SEQ ID NO: 22:
SEQ ID NO: 22 (anti-CD16 single-domain antibody c21; CDRs underlined): 
EVQLVESGGELVQXGGSLRLSCAASGLTFSSYNMGWFRRAPGKEREFVASITWSGRD
TFYADSVKGRFTISRDNAKNTVYLQMSSLKPEDTAVYYCAANPWPVAAPRSGTYWG
QGTQVTVSS
 X is A or P.

The first and second binding domain of the antibody of the invention are fused together, and may, or may not, be separated by an amino acid linker, e.g. a GS linker or a Whitlow/218 linker. The linker preferably has a length of 5-30 amino acids, more preferably 5-20, 5-18, 5-15 or 5-12 amino acids. Examples of suitable GS linkers are as follows:
SSGGGGS (SEQ ID NO: 24)
GSGGGS (SEQ ID NO: 25)
GGGGSGGGS (SEQ ID NO: 26)
SSGGGGSGGGS (SEQ ID NO: 27)
GGSGGSGGSGGSGG (SEQ ID NO: 28)

Examples of Whitlow/218 linkers are as follows:
GSTSGSGKPGSGEGS (SEQ ID NO: 29)
GSTSGSGKPGSGEGSTKG (SEQ ID NO: 30)

In a further preferred embodiment, the antibody of the invention comprises a third peptide domain conveying an extended plasma half-life compared to an antibody lacking the third peptide domain. Variants of the antibody of the invention having an extended half-life may also be designated "half-life extended (HLE) antibody", or HLE-CεmXxCD3-BiTE or HLE-CεmXxCD16-BiKE. The third peptide domain can, for example, be an anti-Human-Serum-Albumin, anti-HSA, antibody domain. More preferably, the anti-HSA antibody domain is the anti-HSA nanobody "Alb11", and has or comprises the following amino acid sequence of SEQ ID NO: 31:
SEQ ID NO: 31 (anti-HSA antibody "Alb11"): 
EVQLVESGGGLVQPGNSLRLSCAASGFTFSSFGMSWVRQAPGKGLEWVSSISGSGSDT
LYADSVKGRFTISRDNAKTTLYLQMNSLXPEDTAVYYCTIGGSLSRSSQGTLVTVSS
 X is R or A.

Alternatively, the third peptide domain can, for example, be an Fc domain of an antibody. The Fc (fragment crystallizable) domain or region is the "tail" region of an antibody interacting with cell surface receptors (Fc receptors). Fusion of an Fc domain for extending the half-life of a protein is known to the skilled person (see, e.g, Binder U, Skerra A, 2025, Strategies for extending the half-life of biotherapeutics: successes and complications, Expert Opinion on Biological Therapy, 25:1, 93-118, DOI: 10.1080/14712598.2024.2436094).

The third domain may be directly fused to the second (e.g., CD3 or CD16) binding domain, preferably to the C-terminal end of the second binding domain, or may be separated by a linker sequence, e.g. a GS linker sequence. Useful GS linkers are described above.

In the antibody of the invention, the structure in relation to the first, second and third binding domains is preferably as follows: N-1^{st} binding domain - [1^{st} linker] - 2^{nd} binding domain [2^{nd} linker] - 3^{rd} binding domain-C. The linkers are optional. However, it is preferred that at least the 2^{nd} linker between the 2^{nd} and 3^{rd} binding domain is present, more preferably also the 1^{st} linker is present. In a construct comprising the anti-human CD3 nanobody CD3 3D-H11, the 1^{st} linker can be dispensed with. The antibody may additionally comprise, at the N-Terminus, a signal peptide, e.g. an IgH signal peptide, and, at the C-Terminus, a histidine tag.

In a particular preferred embodiment, the antibody of the invention has one of the following sequences of SEQ ID NO: 33, 35, 37, 39 or 41 (Nb = nanobody; HLE = half-life extended).
SEQ ID NO: 33 (CεmXxCD3-BiTE): 
MGWSCIILFLVATATGAHSMADIVMSQSPSSLAVSVGEKVTMSCKSSQSLLYSSNQKN
YLAWYQQKPGQSPKLLIYWASTRESGVPDRFTGSGSGTDFTLTISSVKAEDLAVYYCQ
QYYSYPWTFGGGTKLEIKRGSTSGSGKPGSGEGSDVQLQESGPGLVKPSQSLSLTCSVT
GYSITSGYYWNWIRQFPGNKLEWMGSISYDGSNNYNPSLKNRISITRDTSKNQFFLKL
NSVTTEDTATYYCARDYGGFDYWGQGTTLTVSSAAASSGGGGSDIKLQQSGAELARP
GASVKMSCKTSGYTFTRYTMHWVKQRPGQGLEWIGYINPSRGYTNYNQKFKDKATL
TTDKSSSTAYMQLSSLTSEDSAVYYCARYYDDHYCLDYWGQGTTLTVSSVEGGSGGS
GGSGGSGGVDDIQLTQSPAIMSASPGEKVTMTCRASSSVSYMNWYQQKSGTSPKRWI
 
YDTSKVASGVPYRFSGSGSGTSYSLTISSMEAEDAATYYCQQWSSNPLTFGAGTKLEL
KHHHHHH
SEQ ID NO: 35 (CεmXxCD3-HLE-BiTE): 
MGWSCIILFLVATATGAHSMADIVMSQSPSSLAVSVGEKVTMSCKSSQSLLYSSNQKN
YLAWYQQKPGQSPKLLIYWASTRESGVPDRFTGSGSGTDFTLTISSVKAEDLAVYYCQ
QYYSYPWTFGGGTKLEIKRGSTSGSGKPGSGEGSDVQLQESGPGLVKPSQSLSLTCSVT
GYSITSGYYWNWIRQFPGNKLEWMGSISYDGSNNYNPSLKNRISITRDTSKNQFFLKL
NSVTTEDTATYYCARDYGGFDYWGQGTTLTVSSAAASSGGGGSDIKLQQSGAELARP
GASVKMSCKTSGYTFTRYTMHWVKQRPGQGLEWIGYINPSRGYTNYNQKFKDKATL
TTDKSSSTAYMQLSSLTSEDSAVYYCARYYDDHYCLDYWGQGTTLTVSSVEGGSGGS
GGSGGSGGVDDIQLTQSPAIMSASPGEKVTMTCRASSSVSYMNWYQQKSGTSPKRWI
YDTSKVASGVPYRFSGSGSGTSYSLTISSMEAEDAATYYCQQWSSNPLTFGAGTKLEL
KGSGGGSEVQLVESGGGLVQPGNSLRLSCAASGFTFSSFGMSWVRQAPGKGLEWVSS
ISGSGSDTLYADSVKGRFTISRDNAKTTLYLQMNSLRPEDTAVYYCTIGGSLSRSSQGT
LVTVSS
SEQ ID NO: 37 (CεmXxCD16-HLE-BiKE): 
MGWSCIILFLVATATGAHSMADIVMSQSPSSLAVSVGEKVTMSCKSSQSLLYSSNQKN
YLAWYQQKPGQSPKLLIYWASTRESGVPDRFTGSGSGTDFTLTISSVKAEDLAVYYCQ
QYYSYPWTFGGGTKLEIKRGSTSGSGKPGSGEGSDVQLQESGPGLVKPSQSLSLTCSVT
GYSITSGYYWNWIRQFPGNKLEWMGSISYDGSNNYNPSLKNRISITRDTSKNQFFLKL
NSVTTEDTATYYCARDYGGFDYWGQGTTLTVSSAAAGGGGSGGGSEVQLVESGGEL
VQPGGSLRLSCAASGLTFSSYNMGWFRRAPGKEREFVASITWSGRDTFYADSVKGRFT
ISRDNAKNTVYLQMSSLKPEDTAVYYCAANPWPVAAPRSGTYWGQGTQVTVSSGGG
GSGGGSEVQLVESGGGLVQPGNSLRLSCAASGFTFSSFGMSWVRQAPGKGLEWVSSIS
GSGSDTLYADSVKGRFTISRDNAKTTLYLQMNSLRPEDTAVYYCTIGGSLSRSSQGTL
VTVSS
SEQ ID NO: 39 (CεmXxCD3-Nb-BiTE): 
MGWSCIILFLVATATGAHSMADIVMSQSPSSLAVSVGEKVTMSCKSSQSLLYSSNQKN
YLAWYQQKPGQSPKLLIYWASTRESGVPDRFTGSGSGTDFTLTISSVKAEDLAVYYCQ
QYYSYPWTFGGGTKLEIKRGSTSGSGKPGSGEGSDVQLQESGPGLVKPSQSLSLTCSVT
GYSITSGYYWNWIRQFPGNKLEWMGSISYDGSNNYNPSLKNRISITRDTSKNQFFLKL
NSVTTEDTATYYCARDYGGFDYWGQGTTLTVSSAAAEVQLVESGGGLVQPGGSLRLS
CAASGFTFDDYGMSWVRQAPGKWLEWVSDISWNGGSTYYADSVKGRFTISRDNAEN
TLYLQMNSLKPDDTAVYYCAKMGEGGWGANDYWGQGTQVTVSSGSHHHHHH
SEQ ID NO: 41 (CεmXxCD3-Nb-HLE-BiTE):

MGWSCIILFLVATATGAHSMADIVMSQSPSSLAVSVGEKVTMSCKSSQSLLYSSNQKN
YLAWYQQKPGQSPKLLIYWASTRESGVPDRFTGSGSGTDFTLTISSVKAEDLAVYYCQ
QYYSYPWTFGGGTKLEIKRGSTSGSGKPGSGEGSDVQLQESGPGLVKPSQSLSLTCSVT
GYSITSGYYWNWIRQFPGNKLEWMGSISYDGSNNYNPSLKNRISITRDTSKNQFFLKL
NSVTTEDTATYYCARDYGGFDYWGQGTTLTVSSAAAEVQLVESGGGLVQPGGSLRLS
CAASGFTFDDYGMSWVRQAPGKWLEWVSDISWNGGSTYYADSVKGRFTISRDNAEN
TLYLQMNSLKPDDTAVYYCAKMGEGGWGANDYWGQGTQVTVSSGSGGGSEVQLVE
SGGGLVQPGNSLRLSCAASGFTFSSFGMSWVRQAPGKGLEWVSSISGSGSDTLYADSV
KGRFTISRDNAKTTLYLQMNSLRPEDTAVYYCTIGGSLSRSSQGTLVTVSS

The antibody of the invention is a multispecific, preferably a bispecific or trispecific antibody. The skilled person is familiar with, for example, bispecific antibodies and methods for their synthesis (see, for example, Voigt et al. 2022).

The antibody of the invention is suitable for use in a therapy of an IgE-associated disease. In a preferred embodiment, the antibody of the invention is an antibody for use in an anti-allergy therapy. In further preferred embodiments, the antibody of the invention is an antibody for use in a IgE-associated cancer therapy, in particular, a therapy against IgE multiple myeloma, non-Hodgkin lymphoma, or other neoplasm. In a further preferred embodiment, the antibody of the invention is an antibody for use in chronic urticaria.

In a second aspect, the invention relates to a pharmaceutical composition or dosage form for use in a therapy of an IgE-associated disease the pharmaceutical composition comprising an antibody for use in an anti-allergy therapy as described above, and a pharmaceutically acceptable carrier. The composition may, for example, be soluble or in liquid form for parenteral, e.g., intravenous, administration.

In a particular preferred embodiment of the pharmaceutical composition or dosage form of the invention, the pharmaceutical composition or dosage form is for use in an anti-allergy therapy.

In a third aspect, the invention also relates to a nucleic acid encoding an antibody of the invention for use in an anti-allergy therapy described above.

In a preferred embodiment, the nucleic acid according to the invention has or comprises one of the sequences of SEQ ID NO: 32, 34, 36, 38, or 40.

In a further aspect, the invention relates to an anti-allergy therapeutic method wherein an antibody of the invention described above is administered to a subject in need of such a therapy in a therapeutically effective amount.

In a preferred embodiment of the therapeutic method of the invention, an antibody of the invention is administered to the patient intravenously. In another embodiment, the antibody of the invention is administered to the patient by another parenteral route. In embodiments of the therapeutic method of the invention, an antibody of the invention is administered more than once, for example, two, three, four or more times intermittently, e.g., every 7 or 14 days, preferably parenterally. In other embodiments of the therapeutic method of the invention, an antibody of the invention is administered continuously over 1, 2, 3, 4, 5 or more hours or even several days, preferably parenterally.

The antibody of the invention may be administered as a single agent or in combination with one or more other agents. In this regard, the antibody may be administered simultaneously, before or after the other agent or agents.

In order to avoid unnecessary redundancy, and for clarity reasons, it is to be noted that the invention covers any anti-allergy therapeutic method, wherein any of the embodiments of the antibody described above is administered to a subject in need of such a therapy in a therapeutically effective amount.

The invention is explained in more detail below with reference to the accompanying figures and exemplary embodiments for illustrative purposes only.

### BRIEF DESCRIPTION OF THE DRAWINGS

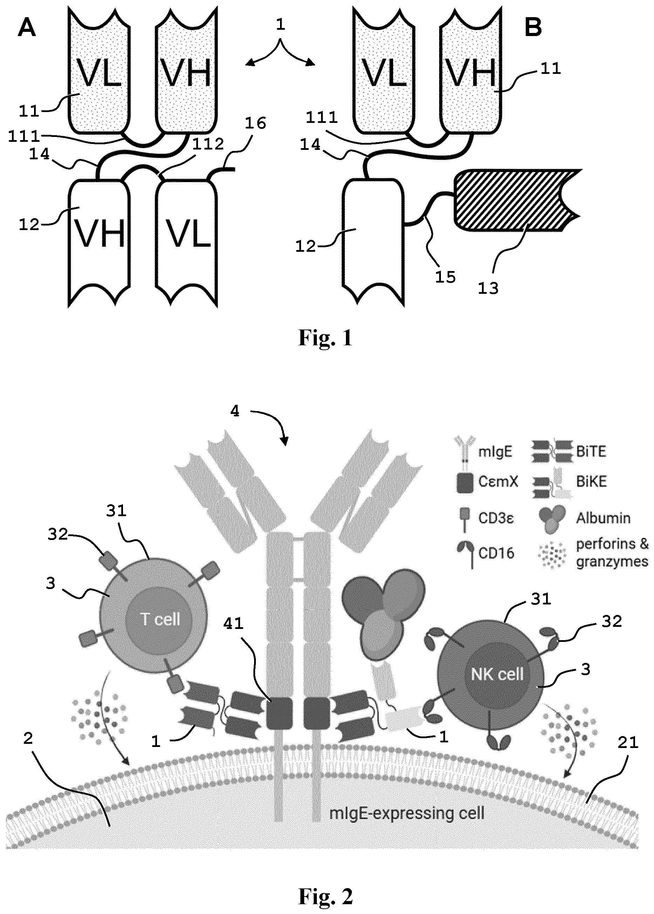

FIG. 1 Schematic drawing showing preferred embodiments of an antibody of the invention. A. Example of a bispecific BiTE of the invention. B. Example of a trispecific BiKE of the invention.
FIG.2 Schematic drawing exemplary depicting the basic concept of the invention.

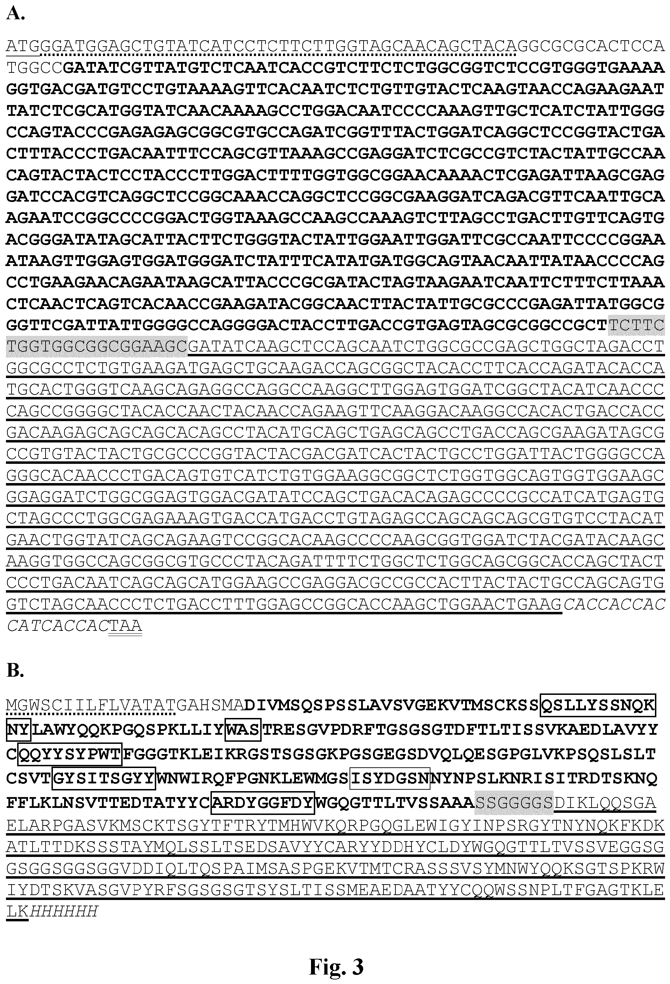

FIG. 3 Nucleotide sequence (A) and amino acid sequence (B) of an embodiment of an antibody of the invention designated CεmXxCD3-BiTE (or 2E3E10-BiTE). A. nucleotide sequence (SEQ ID NO: 32); amino acid sequence (SEQ ID NO: 33); simple underline = Start codon; double underline: stop codon; dotted underline = IgH signal peptide; bold = anti-CεmX domain (2E3E10-scFv VL-VH); gray background = GS linker; bold underlined = anti CD3 domain (here anti CD3 Blinatumomab VH-VL); italic = His tag.

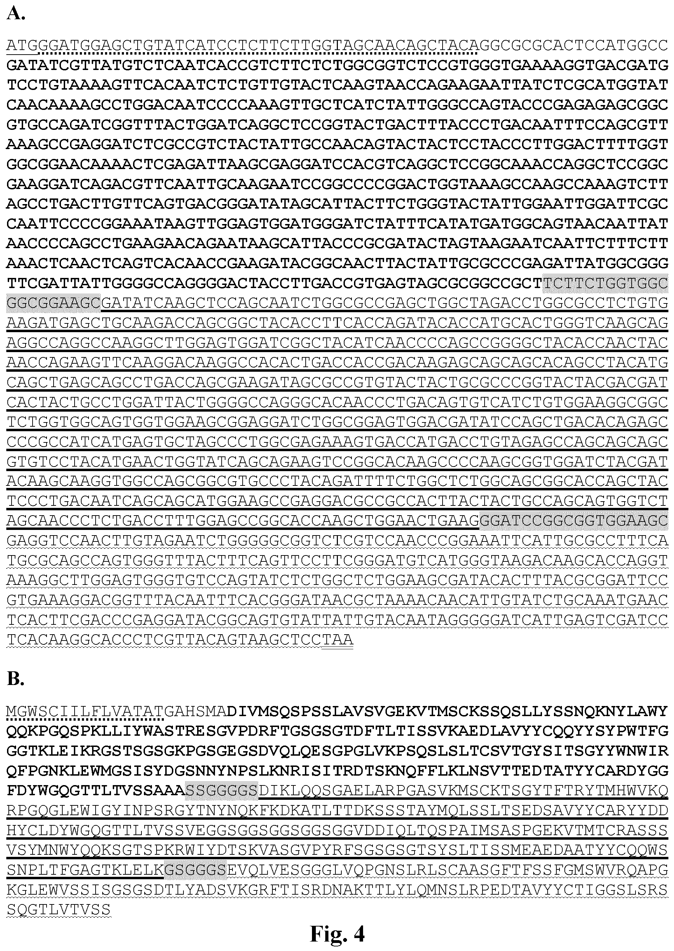

FIG. 4 Nucleotide sequence (A) and amino acid sequence (B) of an embodiment of an antibody of the invention designated CεmXxCD3-HLE-BiTE (or 2E3E10-HLE-BiTE). A. nucleotide sequence (SEQ ID NO: 34); amino acid sequence (SEQ ID NO: 35); simple underline = Start codon; double underline: stop codon; dotted underline = IgH signal peptide; bold = anti-CεmX domain (2E3E10-scFv VL-VH); gray background = GS linker; bold underline = anti CD3 domain (here anti CD3 Blinatumomab VH-VL); wavy underline = HSA nanobody Alb11.

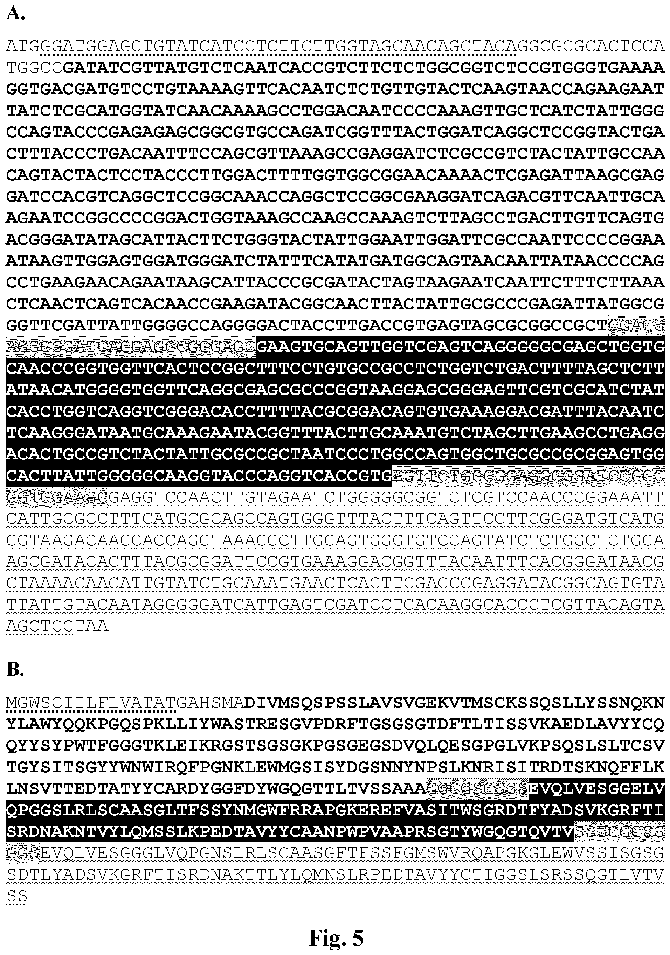

FIG. 5 Nucleotide sequence (A) and amino acid sequence (B) of an embodiment of an antibody of the invention designated CεmXxCD16-Nb-HLE-BiKE (or 2E3E10-HLE-BiKE). A. nucleotide sequence (SEQ ID NO: 36); amino acid sequence (SEQ ID NO: 37); simple underline = Start codon; double underline: stop codon; dotted underline = IgH signal peptide; bold = anti-CεmX domain (2E3E10-scFv VL-VH); gray background = GS linker; inverted letters (white letters on black background) = anti CD16 domain (here anti-human CD16 nanobody c21); wavy underline = HSA nanobody Alb11.

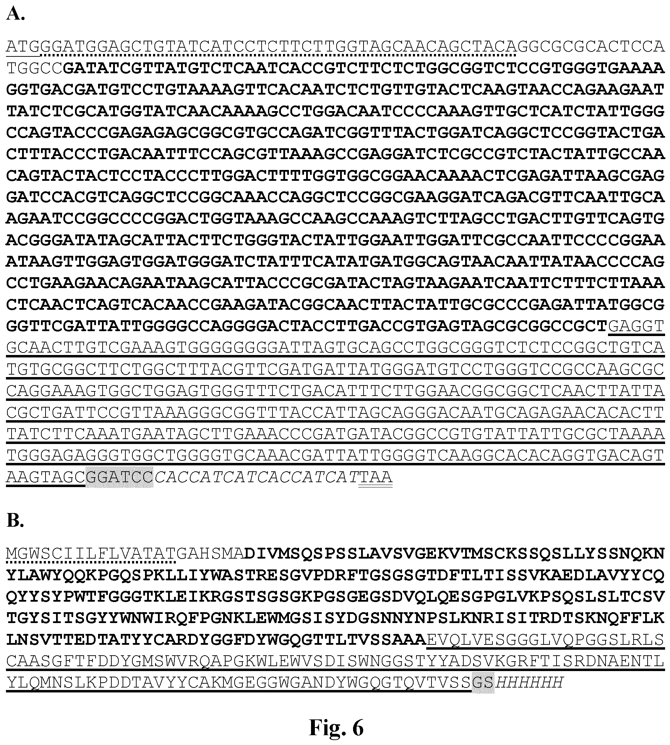

FIG. 6 Nucleotide sequence (A) and amino acid sequence (B) of an embodiment of an antibody of the invention designated CεmXxCD3-Nb-BiTE (or 2E3E10-anti-human-CD3-Nb-BiTE). A. nucleotide sequence (SEQ ID NO: 38); amino acid sequence (SEQ ID NO: 39); simple underline = Start codon; double underline: stop codon; dotted underline = IgH signal peptide; bold = anti-CεmX domain (2E3E10-scFv VL-VH); gray background = GS linker; bold underline = anti CD3 domain (here anti-human CD3 nanobody CD3 3D-H11); italic = His tag.

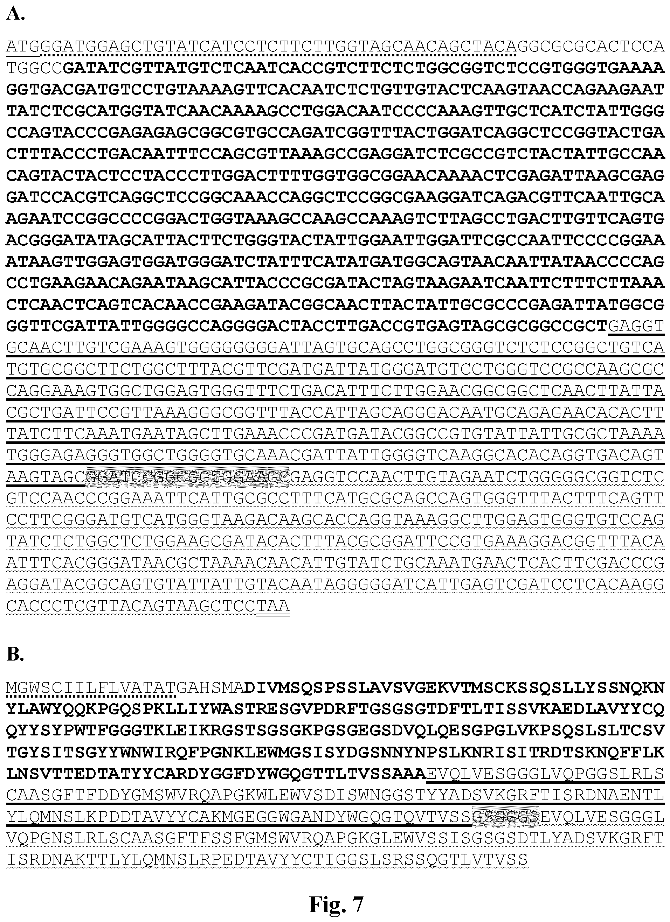

FIG. 7 Nucleotide sequence (A) and amino acid sequence (B) of an embodiment of an antibody of the invention designated CεmXxCD3-Nb-HLE-BiTE (or 2E3E10-anti-human-CD3-Nb-HLE-BiTE). A. nucleotide sequence (SEQ ID NO: 40); amino acid sequence (SEQ ID NO: 41); simple underline = Start codon; double underline: stop codon; dotted underline = IgH signal peptide; bold = anti-CεmX domain (2E3E10-scFv VL-VH); gray background = GS linker; bold underline = anti CD3 domain (here anti CD3 nanobody CD3 3D-H11); wavy underline = HSA nanobody Alb11.

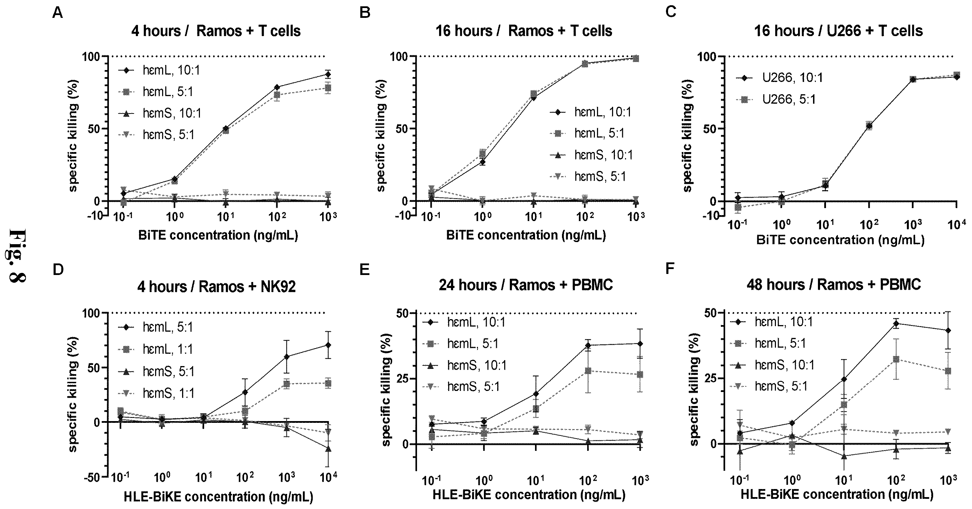

FIG. 8 Graphs showing the calculated specific killing activity (%) mediated by preferred embodiments of antibodies of the invention in various cytotoxicity assays. Cytotoxicity assays were repeated with cells from different donors, cell line batches, and bispecific-engager batches. Ramos^{luc2/eGFP hεmL} ("hεmL"), diamonds and squares, and Ramos^{luc2/eGFP hεmS} ("hεmS"), triangles, were used as target cells in A, B, D-F. U266^{luc2/eGFP}, were used as target cells in C. Labels are structured as follows: target cell line, effector-to-target ratio (A-D) or PBMC-to-target ratio (E,F). Values corresponding to higher E:T ratios are represented in more intense shades of their respective colors. Effector cell types and incubation times are indicated. PBMCs and primary human T cells were from different donors. T cells were expanded for four days before use. All experiments were performed in independent triplicates. Graphs created using GraphPad Prism version 8.0.1 for Windows (GraphPad Software).

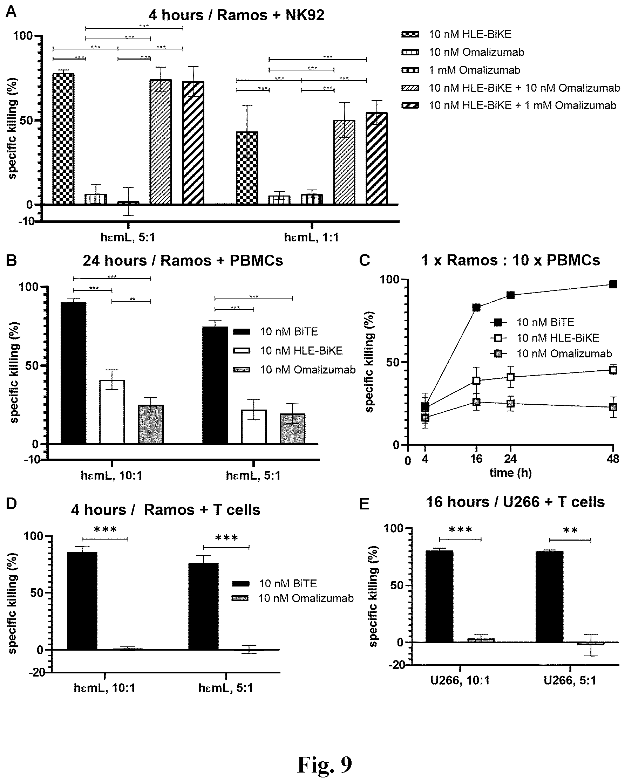

FIG. 9 Calculated specific killing activity (%) mediated by embodiments of the antibody of the invention and Omalizumab in various cytotoxicity assays. Cytotoxicity assays were repeated with cells from different donors, cell line batches, or bispecific engager batches. Ramos^{luc2/eGFP hεmL} ("hεmL") (A-D) and U266^{luc2/eGFP} ("U266") (E) were used as target cells. Value labels depict the antibody concentrations. X-axis labels for A, B, D, and E are structured as follows: target cell line, effector-to-target ratio (A, D, E) or PBMC-to-target ratio (B, C). Experiments depicted in A were performed in independent duplicates, B-E in independent triplicates. PBMCs and primary human T cells were from different donors. The displayed p-values in A + B were calculated using Tukey's multiple comparisons test following a two-way ANOVA. The p-values in D + E were calculated using multiple t-tests. A p-value of < 0.05 is deemed significant (*), a p-value of < 0.01 very significant (**) and a p-value of < 0.001 extremely significant (***). Insignificant p-values of >=0.05 are not displayed. Statistical analyses were performed and graphs were created using GraphPad Prism version 8.0.1 for Windows (GraphPad Software).

Figure 1 schematically shows two embodiments of an antibody 1 of the invention. In the embodiment shown in Fig. 1A, the antibody of the invention 1 is configured as a BiTE, and is composed of two binding domains, a first binding domain 11 (2E3E10 scFv; dotted) binding to CεmX, and a second binding domain 12 binding to an epitope, e.g. an epitope on a surface protein, on the surface of an immune effector cell 3, for example either to CD3, e.g. CD3ε, or to CD16. A His tag 17 is present in this embodiment. Both binding domains 11, 12 are scFv antibody domains composed of the variable light (VL) and heavy chain (VH) antibody domains, connected, in the embodiment shown here, by linkers 111, 112. The linker 111 connecting the VL and VH domains of 2E3E10 scFv can, for example, be a Whitlow/218-Linker (GSTSGSGKPGSGEGS, SEQ ID NO: 29), the linker 112 between the VL and VH domains of the second binding domain 12 a GS linker (e.g. GGSGGSGGSGGSGG, SEQ ID NO: 28). The binding domains 11, 12 are linked by a first linker 14, here a GS linker, with each other. Figure 1B shows an embodiment of the antibody of the invention configured as a BiKE, with three binding domains 11, 12, 13. The first binding domain 11 is again the CεmX binding domain of 2E3E10 scFv (dotted). In the embodiment shown, the second binding domain 12 is configured as a single domain antibody (nanobody, nb) domain. In this embodiment, the second binding domain 12 binds to CD16. The embodiment of an antibody of the invention shown in Fig. 1B comprises a third binding domain 13, binding to albumin. The third domain 13 is part of antibodies of the invention with extended half-life compared to an antibody with only the first and second binding domains 11, 12, i.e. lacking the third binding domain 13. The first and second binding domains 11, 12 are interconnected by a first linker 14, the second and third binding domains 12, 13 are interconnected by a second linker 15, e.g., a GS linker. First, second and third domains 11, 12, 13 are arranged after one another in N-C-terminal direction.

Figure 2 schematically illustrates the basic concept of the invention. A multispecific antibody 1 binds, with its first binding domain (paratope) 11, to the CεmX (EMPD) domain 41 of membrane-bound immunoglobulin E (mIgE) 4 on the surface 21 of an mIGe-expressing target cell 2, e.g., a B cell, and, with its second binding domain (paratope) 12 to a surface protein, e.g., CD3 or CD16, on the surface 31 of an immune effector cell 3, for example a cytotoxic T cell or NK cell. On the left side of the figure, an embodiment of the antibody of the invention configured as a BiTE, engaging T cells as immune effector cell 3, is shown, on the right-hand side of the figure an embodiment of the antibody of the invention configured as a BiKE engaging natural killer cells (NK cells) as immune effector cell 3, is shown. The BiKE is configured as a half-life extended (HLE) variant (see figure 1B) with an albumin-binding third domain 13. In each case, the antibodies of the invention engage an immune effector cell 3, either a T cell or an NK cell, and an mIgE-expressing target cell 2, leading to apoptosis of the target cell 2, e.g. by the excretion of perforins and/or granzymes by the immune effector cell 3.

Figures 3 to 7 show coding DNA sequences (A) and amino acid sequences (B) of preferred embodiments of antibodies of the invention. The antibodies all have the anti-CεmX scFv binding domain of antibody 2E3E10 binding to membrane-bound IgE in common, and are either configured to further bind to CD3 (CD3ε) on T cells or to bind to CD16 on NK cells.

**Table 1. List of exemplary embodiments of an antibody of the invention.**

| **Figure #** | **Designation** | **SEQ ID NO:** | **Description** |
|---|---|---|---|
| 3 | CεmXxCD3-BiTE (2E3E10-BiTE). | 32 (DNA) | T cell engaging BiTE* comprising two scFv antibody domains, an anti CεmX scFv domain and an anti CD3ε scFv antibody domain |
| | | 33 (PRT) | |
| 4 | CεmXxCD3-HLE-BiTE (2E3E10-HLE-BiTE) | 34 (DNA) | Half-life extended (HSA binding) T cell engaging BiTE* of Fig. 3 |
| | | 35 (PRT) | |
| 5 | CεmXxCD16-Nb-HLE-BiKE (E3E10-HLE-BiKE) | 36 (DNA) | Half-life extended (HSA binding) NK cell engaging BiKE* comprising an anti CεmX scFv domain and an anti CD16 single domain antibody (nanobody, nb) domain |
| | | 37 (PRT) | |
| 6 | CεmXxCD3-Nb-BiTE (2E3E10-anti-human-CD3-Nb-BiTE) | 38 (DNA) | T cell engaging BiTE* comprising an anti CεmX scFv domain and an anti-CD3 single domain antibody (nanobody, nb) domain |
| | | 39 (PRT) | |
| 7 | CεmXxCD3-Nb-HLE-BiTE (2E3E10-anti-human-CD3-Nb-HLE-BiTE) | 40 (DNA) | Half-life extended (HSA binding) T cell engaging BiTE of Fig. 6 |
| | | 41 (PRT) | |

| | | | |
|---|---|---|---|
| * The terms "BiTE" and "BiKE" are used here also for embodiments having a third binding specificity, namely a specificity against human serum albumin (HSA), besides the two binding specificities against CεmX and CD3 or CD16. | | | |

### EXAMPLES

### Methods

### Production of BiTE and BiKE Constructs

A codon-optimized version of the complementary deoxyribonucleic acid (cDNA) of anti-CεmX scFv 2E3E10 (US 2018/0022826 A1) was synthesized de novo. The coding sequence of the anti-CD3ε-scFv derived from the murine mAb L2K (Micromet) (Nagorsen et al. 2012), which is also used in Blinatumomab, was obtained from drugbank.com (DrugbankOnline, 2021; DrugBank ID: DB09052) and codon-optimized and de-novo synthesized.

For the production of the HLE-BiKE, the 2E3E10-scFv was cloned into the pCSE2.5-WF211-HLE-BiKE expression plasmid (Hambach et al. 2022) replacing the WF211 nanobody. The expression plasmid already included the CD16-specific nanobody c21 (Behar et al., 2008) and the human-serum-albumin-specific nanobody Alb11 (WO 2012/175400 A1). For the production of the BiTE, the CD16-specific nanobody and the albumin-specific nanobody downstream of the 2E3E10-scFv were replaced by the anti-CD3ε-scFv and a His-tag. In both constructs, the antibodies or nanobodies were connected via Glycine-Serine (GGGGS) linkers. All cloning steps were verified by test digestions and Sanger sequencing.

The resulting plasmids were used to transiently transfect HEK 293-6E cells as previously described (Tom et al., 2008; Jäger et al., 2013; Hambach et al. 2022). Supernatants were collected 6 to 8 days post-transfection. The resulting antibody constructs were purified by affinity chromatography, utilizing HIS-Select Nickel Affinity Gel (Sigma) for the BiTE and protein A sepharose (GE Healthcare) for the HLE-BiKE. Desalting was performed using PD-10 columns (GE Healthcare) and the resulting constructs were subsequently concentrated using Amicon Ultra centrifugal filter units (Merck Millipore, SigmaAldrich) with a molecular weight cut-off of 30 kDa.

The purity of the produced antibody was assessed by SDS-PAGE followed by staining with InstantBlue Coomassie Protein Stain (Abcam). Protein concentration was determined using a BCA test (Pierce BCA Protein Assay Kit, Thermo Scientific). After purification, the antibody constructs were stored in Dulbecco's Phosphate-Buffered Saline (DPBS, Gibco) at 4°C.

### Isolation, Storage and Culture of Peripheral Blood Mononuclear Cells (PBMCs)

PBMCs were isolated from fresh BC using Ficoll density gradient centrifugation and subsequently cryopreserved in foetal calf serum (FCS; Sigma) supplemented with 10% dimethyl sulfoxide. Upon thawing, PBMCs were cultured in RPMI culture medium at a cell density of around 2 × 10⁶ cells/mL and incubated at 37°C with 5% CO₂ and 95% relative humidity. RPMI culture medium consisted of RPMI-1640 (Gibco) supplemented with 10% FCS (Sigma), 100 U/mL penicillin (Gibco) and 100 µg/mL streptomycin (Gibco).

### T Cell Culture

To obtain T cells, PBMCs were resuspended in T cell medium at a cell density of 2 × 10⁶ cells/mL following the thawing steps described above. T cell medium consisted of TexMACS medium (Miltenyi Biotec) supplemented with 1 mM sodium pyruvate (Gibco), 0.05 mM β-mercaptoethanol (Gibco), 3.5% human serum, 20 U/mL IL-2 (Miltenyi Biotec), 100 U/mL penicillin (Gibco) and 100 µg/mL streptomycin (Gibco). The cells were then incubated at 37°C with 5% CO₂ and 95% relative humidity. PBMCs were expanded using human T cell TransAct (Miltenyi Biotec) according to the manufacturer's instructions for 4 days. The T cell medium was changed every 2 days, and cells were split as necessary to maintain a cell density of approximately 2 x 10⁶ cells/mL.

### Target Cell and NK Cell Culture

Cells from the human Burkitt's lymphoma cell line Ramos, stably expressing firefly luciferase (luc2), enhanced green fluorescent protein (eGFP) and the heavy chain of either the short version of mIgE, containing εₛₕₒᵣₜ (Ramos^{luc2/eGFP} hεmS) or the long version of mIgE containing ε_{long} and therefore the CεmX domain (Ramos^{luc2/eGFP} hεmL), were used as target cells (Cichutek 2022). Ramos cells were cultured in suspension cell culture flasks using RPMI culture medium (s. above), which was changed every 2-3 days. The cells were split as necessary to maintain a cell density of 0.3-1.0 × 10⁶ cells per mL.

The U266 cell line, a B-lymphocyte cell line expressing mIgE at a very low antigen density (Nilsson et al., 1970; Brightbill et al., 2010) and also secreting IgE (Ikeyama et al., 1986), was transduced to stably express luc2 and eGFP (Cichutek, 2022). The resulting cells were cultured in suspension cell culture flasks using RPMI culture medium. The medium was exchanged every 2 to 3 days. U266 cells were split as needed to maintain a cell density between 0.3-1.0 x 10⁶ cells per mL.

NK92 cells stably expressing human CD16 and with a knock-out of CD38 (Hambach et al., 2022) were used as effector cells. These NK92^{hCD16 CD38-/-} cells were cultured in suspension cell culture flasks using NK cell medium. NK cell medium consisted of Minimum Essential Medium (MEM) α supplemented with 12.5% horse serum, 12.5% FCS (Sigma), 2 mM glutamine (Gibco), 100 U/mL penicillin (Gibco), 100 µg/mL streptomycin (Gibco), 0.1 mM 2-mercaptoethanol (Gibco) and 100 U/mL IL-2 (Miltenyi Biotec). They were maintained at a cell density of 2-5 × 10⁵ cells per mL and cell aggregates were dispersed every 2-3 days by pipetting up and down.

### Luciferase-based Cytotoxicity Assay

Luciferase-expressing target cells were suspended in 50 µL of the respective effector cell medium at a cell density of 2 × 10⁵ cells/mL and pipetted into a white 96-well plate (OptiPlate, PerkinElmer). A 10-µL mixture of the antibody construct diluted in DPBS was then added to the wells, with each condition tested in triplicate. Wells containing 10 µL of DPBS without the antibody construct served as a control.

Five minutes after the addition of the antibody constructs, effector cells suspended in 50 µL of their respective effector cell medium at varying concentrations to ensure different effector:target (E:T) ratio were added to the wells (t = 0 h).

The plate was then incubated at 37°C with 5% CO₂ for 4, 16, 24, or 48 hours, depending on the specific experiment. Twenty minutes before the desired readout, 50 µL of effector cell medium containing 150 µg/mL D-luciferin (Biosynth) were added to each well. Maximum killing (Killmax) was achieved by lysing the target cells with 1% Triton X-100 shortly before adding the D-luciferin containing medium.

After an additional 20-minute incubation, the bioluminescence intensity (BLI) of the luciferase-expressing cells was measured using a bioluminescence reader (Infinite 200 PRO, Tecan) and expressed as counts per second (cps).

Analyses of the cytotoxicity assay data was performed using Microsoft Excel (Microsoft) and GraphPad Prism version 8.0.1 for Windows (GraphPad Software).

The percentage of antibody construct-mediated killing was calculated as follows: $\begin{matrix}Killing \left(%\right) \\ = \left(1-\left(\frac{mean BLI with antibody \left[cps\right] - mean BLI Killmax \left[cps\right]}{mean BLI control without antibody \left[cps\right] - mean BLI Killmax \left[cps\right]}\right)\right) ∗ 100\end{matrix}$

To test for possible interference of human serum albumin (HSA), MEM α supplemented with 100 U/mL IL-2 (Miltenyi Biotec) + 100 U/mL penicillin (Gibco) + 100 µg/mL streptomycin (Gibco) was used instead of effector cell medium. This medium was prepared with either no HSA or 4.45 g/L HSA (CSL Behring GmbH) added.

### Albumin Binding of HLE-BiKE

The NK92 cells were incubated with varying concentrations of HLE-BiKE for 20 min at 4°C. After washing, a biotinylated human albumin (Abcam) was added and the cells were incubated for another 20 min at 4°C. Following a second washing step REAfinity Biotin-PE (Miltenyi Biotec) was added. Control samples were stained without HLE-BiKE or human albumin-streptavidin conjugate. After a final incubation and washing step, the cells were analyzed via flow cytometry (LSRFortessa, BD Biosciences).

### Analysis of donor variability

To characterize the buffy coats, flow cytometry (LSRFortessa, BD Biosciences) was employed. PBMCs from different donors were thawed and cultured in RPMI culture medium for one day. During preparation and staining, cells were washed with FACS buffer containing PBS + 0.2% bovine serum albumin (BSA; Sigma). Antibodies and reagents (Table 1) were used according to the manufacturers' protocols. Data was analyzed using FACSDiva (BD Biosciences) software.

**Table 2: Antibodies and reagents used for the analysis of donor variability. The table lists the target antigen, fluorochrome, clone, and manufacturer for each antibody, as well as the viability dye used.**

| Target | Fluorochrome | Clone | Manufacturer |
|---|---|---|---|
| CD3 | Pacific Blue | UCHT1 | DB Pharmingen |
| CD4 | VioGreen | REA623 | Miltenyi Biotec |
| CD8 | APC-Vio 770 | REA734 | Miltenyi Biotec |
| CD14 | APC | REA599 | Miltenyi Biotec |
| CD16 | FITC | CLB/fcGran 1 | BD Biosciences |
| CD19 | PE-Vio 770 | REA675 | Miltenyi Biotec |
| CD56 | PE | REA196 | Miltenyi Biotec |
| Live/Dead | 7-AAD | -- | Miltenyi Biotec |

### Analysis of T-cell activation

To assess the T-cell activation mediated by the BiTEs, PBMCs thawed 3 days prior and maintained in PBMC medium were incubated at a cell density of 10⁶/mL for 2 days under different conditions: without any additive; with an anti-CD3/anti-CD28 reagent (TransAct, Miltenyi Biotec) according to the manufacturer's protocol as a positive control; with Ramos-hεmL; with 400 ng/mL BiTE; with 400 ng/mL BiTE and Ramos-hεmL and with 400 ng/mL BiTE and Ramos-hεmS. Ramos cells were added to achieve a final cell density of 10⁶ cells/mL. After 2 days of incubation, cells were washed and incubated for 20 min in 20% autologous plasma. The cells were then washed twice and stained with the premixed antibody cocktail (Table 3). Following a 20-minute incubation at approximately 22 °C in the dark, the cells were washed once more, stained with 7-aminoactinomycin D (7-AAD; Miltenyi Biotec) according to the manufacture's protocol and analysed by flow cytometry (LSRFortessa, BD Biosciences). UltraComb eBeads Plus (Invitrogen) were used for compensation, with 7-AAD compensation performed using only 7-AAD stained cells. Data were analysed using FACSDiva software (BD Biosciences).

**Table 3: Antibodies used in the premixed antibody cocktail for the analysis of T cell activation. The table lists the target antigen, fluorochrome, clone, and manufacturer for each antibody.**

| Target | Fluorochrome | Clone | Manufacturer |
|---|---|---|---|
| CD3 | Pacific Blue | UCHT1 | DB Pharmingen |
| CD4 | VioGreen | REA623 | Miltenyi Biotec |
| CD8 | APC-Vio770 | REA734 | Miltenyi Biotec |
| CD69 | PE | FN50 | BioLegend |
| CD25 | PE-Vio770 | REA570 | Miltenyi Biotec |
| CD107a | AF-647 | eBioH4A3 | eBioscience |

### Results

### BiTE & HLE-BiKE specifically and effectively mediate the killing of Ramos cells expressing the CεmX domain

The above-mentioned antibodies of the invention designated "CεmXxCD3-BiTE" (2E3E10-BiTE) and CεmXxCD16-Nb-HLE-BiKE (E3E10-HLE-BiKE), see table 1, were investigated.

Both bispecific engagers, the BiTE and the HLE-BiKE, are designed around a single-chain variable fragment (scFv), isolated from the antibody 2E3E10, targeting the CεmX domain of mIgE (Ma & Fay, 2017). In the BiTE construct another scFv targeting CD3ε, isolated from antibody L2K, which has already been used in the clinically successful Blinatumomab (Nagorsen et al. 2012; Wu & Cheung 2018), is connected to the 2E3E10 scFv via a Gly-Ser linker (Fig. 1A) (Chen et al., 2013). In the HLE-BiKE construct, the 2E3E10-scFv is connected to a CD16-targeting nanobody (Behar et al., 2008), which is itself connected to a nanobody against human serum albumin (Fig. 1B) (WO 2012/175400 A1).

Following molecular cloning, production and purification, the integrity and purity of our CεmX-targeting BiTE and HLE-BiKE were confirmed using SDS-PAGE (not shown). To assess the functionality of the BiTE and HLE-BiKE, we utilized luciferase-based cytotoxicity assays. Ramos^{luc2/eGFP} cells expressing the mIgE heavy chain either with (hεmL) or without (hεmS) the targeted CεmX domain were used as target and control cells, respectively. For simplicity, the Ramos^{luc2/eGFP-hεmL} cells will be referred to as "hεmL", and the Ramos^{luc2/eGFP-hεmS} cells will be referred to as "hεmS" in the following. For BiTE testing, Ramos cells were co-incubated with primary human T cells at E:T ratios of 10: 1 and 5:1. The BiTE was added at various concentrations ranging from 0.1 ng/mL to 1,000 ng/mL.

The results demonstrated that the BiTE effectively mediated killing of hεmL cells, which express the CεmX domain, while sparing hεmS cells, which lack the CεmX domain The efficacy of the BiTE was dose-dependent, with measurable cytotoxicity at concentration as low as 1 ng/mL (Fig. 8A, B).

The efficacy of the HLE-BiKE was assessed using analogous luciferase-based cytotoxicity assays employing NK92^{hCD16 CD38-/-} cells, hereafter referred to as "NK92", and primary human PBMCs as effector cells. Similarly to the BiTE, the HLE-BiKE specifically and effectively mediated the killing of CεmX-expressing cells in a dose- and E:T ratio-dependent manner (Fig. 8D-F). Compared to the BiTE construct, a higher HLE-BiKE concentration of 10 ng/mL was required to mediate measurable killing. Additionally, the HLE-BiKE exhibited a slightly lower percentage of calculated specific killing compared to the BiTE at comparable E:T ratios, time points and antibody concentration. This difference may be partially explained by the general cytotoxic activity of the NK92 cells even in the absence of the HLE-BiKE, leading to high non-specific killing in the controls.

In the cytotoxicity assays with primary human PBMCs, PBMC-to-target ratios of 10: 1 and 5: 1 were used. This translated to effective E:T ratios of up to approximately 2: 1, due to the low percentage of CD16+ cells and even lower numbers of NK cells in the PBMC samples. Despite this, the HLE-BiKE still specifically mediated the killing of hεmL cells, proving that the HLE-BiKE is effective not only with engineered NK92 cells, but also with primary human NK cells and potentially other CD16-bearing cells (Fig. 8E, F).

### Further characterization of the HLE-BiKE and its HSA-binding properties

We further characterized the HLE-BiKE, focusing on the HSA-binding nanobody. We first performed albumin binding assays and showed that the HLE-BiKE can simultaneously bind to human CD16 and HSA (not shown). We further tested whether the presence of HSA in vitro affects the killing efficacy of the HLE-BiKE. Cytotoxicity assays with added HSA revealed that the presence of HSA did not impact the killing efficacy of the HLE-BiKE in vitro (not shown). This result is encouraging as it suggests that the HLE-BiKE might function effectively in physiological conditions where HSA is abundant.

### BiTE mediates the killing of low-antigen-density cells

After confirming the functionality of the BiTE construct, we aimed to test its efficacy on cells with a low antigen density. Therefore, we used cells from the U266 myeloma cell line, which express mIgE at very low levels (Nilsson et al. 1970; Batista et al. 1996; Ward et al. 2018). Despite the low mIgE density, the BiTE effectively mediated the killing of U266 cells by primary human T cells (Fig. 8C). This result is particularly promising, as IgE-class switched B cells are believed to downregulate mIgE upon differentiation into antibody-secreting plasma cells (Achatz et al., 2008). In contrast to the promising efficacy of the BiTE, cytotoxicity assays on U266 cells using the HLE-BiKE and NK92 cells as effector cells did not show specific killing activity.

Despite extensive efforts, it was technically not possible to accurately detect depletion of the very rare mIgE-positive cells in peripheral blood in an in-vitro setting, a known challenge previously noted by multiple other groups (Gauvreau et al. 2014; Harris et al. 2016; Eckl-Dorna et al. 2019).

### BiTE induces robust T cell activation only in the presence of target cells

To investigate T cell activation, we conducted co-culture experiments using PBMCs and analysed the results via flow cytometry. Our findings revealed that the T cell activation markers CD25 and CD69 were highly expressed on both CD4+ and CD8+ PBMCs when co-cultured together with BiTE and hεmL target cells. In contrast, only slight expression of these markers could be observed when PBMCs were incubated with BiTE or target cells alone (not shown). These results demonstrate that the BiTE induces polyclonal and major-histocompatibility-complex (MHC-) independent T cell activation of both CD4+ and CD8+ T cells only when both, BiTE and target cells are present. The level of activation, as indicated by the expression of activation markers, was stronger than that induced by the T-cell stimulating reagent TransAct (Miltenyi Biotec). Importantly, these results also indicate that the BiTE construct of the invention promotes highly effective and robust T cell activation only in the presence, but not in the absence of target cells.

### Comparative analysis and characterization of BiTE, HLE-BiKE and Omalizumab

In a further set of experiments, the efficacy of constructs of the invention was evaluated in comparison to and in combination with Omalizumab. First, a 4-h cytotoxicity assay utilizing NK92 cells was performed and superior ability of the HLE-BiKE of the invention to mediate killing of hεmL cells compared to Omalizumab was found. Notably, the presence of Omalizumab did not affect the ability of the HLE-BiKE of the invention to mediate killing when both were administered together (Fig. 9A).

Next, the constructs of the invention were compared to Omalizumab, all at the same concentration of 10 nM, using unstimulated PBMCs as effector cells in 24-h cytotoxicity assays. The results showed that the killing mediated by the BiTE according to the invention was most efficient (Fig. 9B, C). This is likely due to the higher number of T cells compared to NK cells in the PBMCs samples (not shown) and a higher efficacy of the BiTE according to the invention compared to the HLE-BiKE according to the invention at low concentrations. The HLE-BiKE according to the invention still exhibited higher killing compared to Omalizumab, although the difference was less pronounced than in the previously mentioned cytotoxicity assay with NK92 cells (Fig. 9A). Interestingly, Omalizumab showed high killing of the hεmS cells, compared to its cytotoxic activity on hεmL cells (not shown). There are multiple potential explanations for this observation. One possibility is a more effective ADCC due to the Omalizumab binding site being closer to the cell membrane in hεmS cells compared to hεmL cells (Cleary et al. 2017). Another possible explanation could be crosslinking of mIgE leading to apoptosis, which was found to occur nearly exclusively with short mIgE lacking the CεmX domain (Batista et al. 1996; Poggianella et al. 2006; Feichtner et al. 2008). However, this mechanism remains controversial, specifically with regard to Omalizumab (Chan et al. 2013).

In cytotoxicity assays using primary human T cells as effector cells, the BiTE showed significantly superior killing of hεmL cells compared to Omalizumab (Fig. 9D). In these assays, we observed an even greater difference in the killing activity of Omalizumab on hεmS cells compared to hεmL cells (not shown). Given that the level of cytotoxic activity observed is unlikely to be attributed solely to the cytotoxic activity of the rather small CD16+ T cell subset, including for example natural killer T (NKT) cells, this finding points towards an ADCC-independent mechanism of Omalizumab function. One such mechanism could be the aforementioned mIgE crosslinking leading to apoptosis. Cytotoxicity assays using BiTE and pre-stimulated T cells on U266 cells with low antigen density, revealed no cytotoxic activity of Omalizumab, likely due to its effector functions depending on higher antigen density (Fig. 9E). In contrast, the BiTE demonstrated high efficacy under this condition, highlighting its ability to mediate robust cytotoxicity even in the context of low antigen density and low BiTE concentrations.

In summary, the results demonstrated the remarkable efficacy of the novel BiTE and HLE-BiKE construct of the invention in mediating specific and effective killing of CεmX-expressing target cells in vitro. The constructs mediate dose- and E:T-ratio dependent cytotoxicity, with the BiTE exhibiting particularly robust activity even at low antigen densities and at BiTE concentrations as low as 1 ng/mL.

### References

Achatz, G. & Lamers, M.C. (1997) In vivo analysis of the cytoplasmic domain of mIgE antibodies. International Archives of Allergy and Immunology, 113(1-3), 142-145; doi.org/10.1159/000237529.
Achatz, G., Lamers, M. & Crameri, R. (2008) Membrane Bound IgE: The Key Receptor to Restrict High IgE Levels. The Open Immunology Journal, 1(1), 25-32; doi.org/10.2174/1874226200801010025.
Akdis, C.A., Akdis, M., Boyd, S.D., Sampath, V., Galli, S.J. & Nadeau, K.C. (2023) Allergy: Mechanistic insights into new methods of prevention and therapy. Science Translational Medicine, 15(679), eadd2563; doi.org/10.1126/scitranslmed.add2563.
Balbino, B., Conde, E., Marichal, T., Starkl, P. & Reber, L.L. (2018) Approaches to target IgE antibodies in allergic diseases. Pharmacology & Therapeutics, 191, 50-64; doi.org/10.1016/j.pharmthera.2018.05.015.
Batista, F.D., Anand, S., Presani, G., Efremov, D.G. & Burrone, O.R. (1996) The two membrane isoforms of human IgE assemble into functionally distinct B cell antigen receptors. Journal of Experimental Medicine, 184(6), 2197-2205; doi.org/10.1084/jem.184.6.2197.
Behar G, Sibéril S, Groulet A, Chames P, Pugnière M, Boix C, Sautès-Fridman C, Teillaud JL, Baty D., Isolation and characterization of anti-FcgammaRIII (CD16) llama single-domain antibodies that activate natural killer cells, 2008, Protein Eng Des Sel. 21(1):1-10, doi: 10.1093/protein/gzm064
Bergmann, K.-C., Heinrich, J. & Niemann, H. (2016) Current status of allergy prevalence in Germany: Position paper of the Environmental Medicine Commission of the Robert Koch Institute. Allergo Journal International, 25(1), 6-10; doi.org/10.1007/s40629-016-0092-6.
Bernstein, D.I., Schwartz, G. & Bernstein, J.A. (2016) Allergic Rhinitis: Mechanisms and Treatment. Immunology and Allergy Clinics of North America, 36(2), 261-278; doi.org/10.1016/j.iac.2015.12.004
Bousquet, J., Anto, J.M., Bachert, C., Baiardini, I., Bosnic-Anticevich, S. & Walter Canonica, G. et al. (2020) Allergic rhinitis. Nature Reviews Disease Primers, 6(1), 95; doi.org/10.1038/s41572-020-00227-0.
Brinkmann U, Kontermann RE, 2017, The making of bispecific antibodies, MAbs. 9(2):182-212, DOI: 10.1080/19420862.2016.1268307.
Brightbill, H.D., Jeet, S., Lin, Z., Yan, D., Zhou, M. & Tan, M. et al. (2010) Antibodies specific for a segment of human membrane IgE deplete IgE-producing B cells in humanized mice. The Journal of Clinical Investigation, 120(6), 2218-2229. doi.org/10.1172/JCI40141.
Burt R, Warcel D, Fielding AK. Blinatumomab, a bispecific B-cell and T-cell engaging antibody, in the treatment of B-cell malignancies. Hum Vaccin Immunother. 2019;15(3):594-602. doi: 10.1080/21645515.2018.1540828
Chan YH, Lee YT, Chou HW, Wu PC, Chen JB, Li CH, Cheng TT, Chen NY, Lue KH, Chang TW; Developing an antibody targeting CεmX of mIgE for the treatment of allergic and other IgE-mediated diseases (VAC6P.954). J Immunol 1 May 2014; 192 (1_Supplement): 140.15. doi.org/10.4049/jimmunol.192.Supp.140.15
Chan, M.A., Gigliotti, N.M., Dotson, A.L. & Rosenwasser, L.J. (2013) Omalizumab may decrease IgE synthesis by targeting membrane IgE+ human B cells. Clinical and Translational Allergy, 3(1), 29. Available from: https://doi.org/10.1186/2045-7022-3-29.
Chen, H.Y., Liu, F.-T., Hou, C.M.H., Huang, J.S.W., Sharma, B.B. & Chang, T.W. (2002) Monoclonal antibodies against the C(epsilon)mX domain of human membrane-bound IgE and their potential use for targeting IgE-expressing B cells. International Archives of Allergy and Immunology, 128(4), 315-324; doi.org/10.1159/000063860.
Chen, J.B., Wu, P.C., Hung, AF-H, Chu CY, Tsai TF, Yu HM, Chang HY, Chang, TW; Unique Epitopes on CεmX in IgE-B Cell Receptors Are Potentially Applicable for Targeting IgE-Committed B Cells. J Immunol 15 February 2010; 184 (4): 1748-1756; doi.org/10.4049/jimmunol.0902437
Chen, X., Zaro, J.L. & Shen, W.-C. (2013) Fusion protein linkers: property, design and functionality. Advanced Drug Delivery Reviews, 65(10), 1357-1369. Available from: https://doi.org/10.1016/j.addr.2012.09.039.
Chu, H-M, Wright, J, Chan, Y-H, Lin, C-J, Chang, TW, Lim, C, (2014), Two potential therapeutic antibodies bind to a peptide segment of membrane-bound IgE in different conformations. Nat. Commun. 5:3139 doi: 10.1038/ncomms4139.
Cichutek, S. (2022) Präklinische Entwicklung adoptiver Immuntherapien für Typ-I-Allergien auf Basis chimärer Antigenrezeptoren (CARs) gegen IgE-positive B-Zellen. Staats- und Universitätsbibliothek Hamburg Carl von Ossietzky.
Cleary, K.L.S., Chan, H.T.C., James, S., Glennie, M.J. & Cragg, M.S. (2017) Antibody Distance from the Cell Membrane Regulates Antibody Effector Mechanisms. Journal of Immunology (Baltimore, Md. : 1950), 198(10), 3999-4011; doi:/10.4049/jimmunol.1601473.
Davies, J.M., Platts-Mills, T.A. & Aalberse, R.C. (2013) The enigma of IgE+ B-cell memory in human subjects. The Journal of Allergy and Clinical Immunology, 131(4), 972-976; doi.org/10.1016/j.jaci.2012.12.1569.
Demaria O, Gauthier L, Debroas G, Vivier E, 2021, Natural killer cell engagers in cancer immunotherapy: Next generation of immuno-oncology treatments, Eur. J. Immunol. 51: 1934-1942, doi:10.1002/eji.202048953;
Durham SR, Shamji MH. Allergen immunotherapy: past, present and future. Nat Rev Immunol. 2023 May;23(5):317-328. doi: 10.1038/s41577-022-00786-1.
Eckl-Dorna, J., Pree, I., Reisinger, J., Marth, K., Chen, K.-W. & Vrtala, S. et al. (2012) The majority of allergen-specific IgE in the blood of allergic patients does not originate from blood-derived B cells or plasma cells. Clinical and Experimental Allergy : Journal of the British Society for Allergy and Clinical Immunology, 42(9), 1347-1355; doi.org/10.1111/j.1365-2222.2012.04030.x.
Eckl-Dorna, J. & Niederberger, V. (2013) What is the source of serum allergen-specific IgE? Current Allergy and Asthma Reports, 13(3), 281-287; doi.org/10.1007/s11882-013-0348-x.
Eckl-Dorna, J., Villazala-Merino, S., Campion, N.J., Byazrova, M., Filatov, A. & Kudlay, D. et al. (2019) Tracing IgE-Producing Cells in Allergic Patients. Cells, 8(9); doi.org/10.3390/cells8090994.
Eckl-Dorna, J., Villazala-Merino, S., Campion, N.J., Byazrova, M., Filatov, A. & Kudlay, D. et al. (2019) Tracing IgE-Producing Cells in Allergic Patients. Cells, 8(9); doi.org/10.3390/cells8090994.
Elshiaty, M., Schindler, H., & Christopoulos, P. (2021). Principles and Current Clinical Landscape of Multispecific Antibodies against Cancer. International Journal of Molecular Sciences, 22(11), 5632; doi.org/10.3390/ijms22115632.
Feichtner, S., Inführ, D., Achatz-Straussberger, G., Schmid, D., Karnowski, A. & Lamers, M. et al. (2008) Targeting the extracellular membrane-proximal domain of membrane-bound IgE by passive immunization blocks IgE synthesis in vivo. Journal of Immunology (Baltimore, Md.: 1950), 180(8), 5499-5505; doi.org/10.4049/jimmunol.180.8.5499.
Felices M, Lenvik TR, Davis ZB, Miller JS, Vallera DA, 2016, Generation of BiKEs and TriKEs to Improve NK Cell-Mediated Targeting of Tumor Cells, Methods Mol Biol. 1441:333-46, DOI: 10.1007/978-1-4939-3684-7_28.
Gasser, P., Tarchevskaya, S.S., Guntern, P., Brigger, D., Ruppli, R. & Zbären, N. et al. (2020) The mechanistic and functional profile of the therapeutic anti-IgE antibody ligelizumab differs from omalizumab. Nature Communications, 11(1), 165; doi.org/10.1038/s41467-019-13815-w.
Gauvreau, G.M., Harris, J.M., Boulet, L.-P., Scheerens, H., Fitzgerald, J.M. & Putnam, W.S. et al. (2014) Targeting membrane-expressed IgE B cell receptor with an antibody to the M1 prime epitope reduces IgE production. Science Translational Medicine, 6(243), 243ra85; doi.org/10.1126/scitranslmed.3008961.
Goebeler, M.-E. & Bargou, R.C. (2020) T cell-engaging therapies - BiTEs and beyond. Nature Reviews. Clinical Oncology, 17(7), 418-434; doi.org/10.1038/s41571-020-0347-5.
Gupta, R.S., Warren, C.M., Smith, B.M., Jiang, J., Blumenstock, J.A. & Davis, M.M. et al. (2019) Prevalence and Severity of Food Allergies Among US Adults. JAMA Network Open, 2(1), e185630; doi.org/10.1001/jamanetworkopen.2018.5630.
Hambach J, Fumey W, Stähler T, Gebhardt AJ, Adam G, Weisel K, Koch-Nolte F, Bannas P. Half-Life Extended Nanobody-Based CD38-Specific Bispecific Killer cell Engagers Induce Killing of Multiple Myeloma Cells. Front Immunol. 2022 May 16;13:838406. doi: 10.3389/fimmu.2022.838406.
Hanania, N.A., Niven, R., Chanez, P., Antoine, D., Pfister, P. & Garcia Conde, L. et al. (2022) Long-term effectiveness and safety of omalizumab in pediatric and adult patients with moderate-to-severe inadequately controlled allergic asthma. The World Allergy Organization Journal, 15(10), 100695; doi.org/10.1016/j.waojou.2022.100695.
Harris, J.M., Maciuca, R., Bradley, M.S., Cabanski, C.R., Scheerens, H. & Lim, J. et al. (2016) A randomized trial of the efficacy and safety of quilizumab in adults with inadequately controlled allergic asthma. Respiratory Research, 17(1), 29; doi.org/10.1186/s12931-016-0347-2.
He, J.-S., Narayanan, S., Subramaniam, S., Ho, W.Q., Lafaille, J.J. & Curotto de Lafaille, M.A. (2015) Biology of IgE production: IgE cell differentiation and the memory of IgE responses. Current Topics in Microbiology and Immunology, 388, 1-19; doi.org/10.1007/978-3-319-13725-4_1.
Hu, J., Chen, J., Ye, L., Cai, Z., Sun, J. & Ji, K. (2018) Anti-IgE therapy for IgE-mediated allergic diseases: from neutralizing IgE antibodies to eliminating IgE+ B cells. Clinical and Translational Allergy, 8(1), 27; doi.org/10.1186/s13601-018-0213-z.
Jardieu, P. (1995) Anti-IgE therapy. Current Opinion in Immunology, 7(6), 779-782; doi.org/10.1016/0952-7915(95)80047-6.
Ikeyama, S., Nakagawa, S., Arakawa, M., Sugino, H. & Kakinuma, A. (1986) Purification and characterization of IgE produced by human myeloma cell line, U266. Molecular Immunology, 23(2), 159-167; doi.org/10.1016/0161-5890(86)90038-6.
Jäger, V., Büssow, K., Wagner, A., Weber, S., Hust, M. & Frenzel, A. et al. (2013) High level transient production of recombinant antibodies and antibody fusion proteins in HEK293 cells. BMC Biotechnology, 13, 52. Available from: https://doi.org/10.1186/1472-6750-13-52.
Jin BK, Odongo S, Radwanska M, Magez S. NANOBODIES®: A Review of Diagnostic and Therapeutic Applications. Int J Mol Sci. 2023 Mar 22;24(6):5994. doi: 10.3390/ijms24065994 Joshi, A.Y., Iyer, V.N., Boyce, T.G., Hagan, J.B., Park, M.A. & Abraham, R.S. (2009) Elevated serum immunoglobulin E (IgE): when to suspect hyper-IgE syndrome-A 10-year pediatric tertiary care center experience. Allergy and Asthma Proceedings, 30(1), 23-27; doi.org/10.2500/aap.2009.30.3193.
Kirak, O. & Riethmüller, G. (2015) A novel, nonanaphylactogenic, bispecific IgE-CD3 antibody eliminates IgE(+) B cells. The Journal of Allergy and Clinical Immunology, 136(3), 800-802.e3; doi.org/10.1016/j.jaci.2015.02.017.
Klein, C., Brinkmann, U., Reichert, J.M. & Kontermann, R.E. (2024) The present and future of bispecific antibodies for cancer therapy. Nature Reviews Drug Discovery, 23(4), 301-319; doi.org/10.1038/s41573-024-00896-6.
Kontermann, R. (2009). Strategies to Extend Plasma Half-Lives of Recombinant Antibodies. BioDrugs : clinical immunotherapeutics, biopharmaceuticals and gene therapy. 23. 93-109. doi: 10.2165/00063030-200923020-00003.
Ling, X.-J., Wei, J.-F. & Zhu, Y. (2023) Aiming to IgE: Drug development in allergic diseases. International Immunopharmacology, 121, 110495; doi.org/10.1016/j.intimp.2023.110495. Ma, Z. & Fay, B.F. (2017) EMPD-specific chimeric antigen receptor mediates T cell cytotoxicity to B cells expressing membrane IgE. The Journal of Immunology, 198(1 Supplement), 194.9-194.9; doi.org/10.4049/jimmunol.198.Supp.194.9
Mocquot P, Mossazadeh Y, Lapierre L, Pineau F, Despas F. The pharmacology of blinatumomab: state of the art on pharmacodynamics, pharmacokinetics, adverse drug reactions and evaluation in clinical trials. J Clin Pharm Ther. 2022 Sep;47(9):1337-1351. doi: 10.1111/jcpt.13741.
Moradi-Kalbolandi, S, Sharifi-K, A, Darvishi, B, Majidzadeh-A K, Jalili, N, Sadeghi, S, Mosayebzadeh, M, Sanati, H, Salehi, M, Farahmand, L, (2020) Evaluation the potential of recombinant anti-CD3 nanobody on immunomodulatory function, Molecular Immunology 118, 174-181, doi.org/10.1016/j.molimm.2019.12.017.
Nagorsen, D., Kufer, P., Baeuerle, P.A. & Bargou, R. (2012) Blinatumomab: a historical perspective. Pharmacology & Therapeutics, 136(3), 334-342; doi.org/10.1016/j.pharmthera.2012.07.013.
Nilsson, K., Bennich, H., Johansson, S.G. & Pontén, J. (1970) Established immunoglobulin producing myeloma (IgE) and lymphoblastoid (IgG) cell lines from an IgE myeloma patient. Clinical and Experimental Immunology, 7(4), 477-489; pubmed.ncbi.nlm.nih.gov/4097745/.
Okayama, Y., Matsumoto, H., Odajima, H., Takahagi, S., Hide, M. & Okubo, K. (2020) Roles of omalizumab in various allergic diseases. Allergology International: Official Journal of the Japanese Society of Allergology, 69(2), 167-177; doi.org/10.1016/j.alit.2020.01.004.
Pelaia, C., Calabrese, C., Terracciano, R., Blasio, F. de, Vatrella, A. & Pelaia, G. (2018) Omalizumab, the first available antibody for biological treatment of severe asthma: more than a decade of real-life effectiveness. Therapeutic Advances in Respiratory Disease, 12, 1753466618810192; doi.org/10.1177/1753466618810192.
Platts-Mills, T.A.E. (2015) The allergy epidemics: 1870-2010. The Journal of Allergy and Clinical Immunology, 136(1), 3-13; doi.org/10.1016/j.jaci.2015.03.048.
Poggianella, M., Bestagno, M. & Burrone, O.R. (2006) The extracellular membrane-proximal domain of human membrane IgE controls apoptotic signaling of the B cell receptor in the mature B cell line A20. Journal of Immunology (Baltimore, Md. : 1950), 177(6), 3597-3605. Available from: https://doi.org/10.4049/jimmunol.177.6.3597.
Rodak, A., Stadlbauer, K., Bobbili, M.R., Smrzka, O., Rüker, F. & Wozniak Knopp, G. (2023) Development of a Cytotoxic Antibody-Drug Conjugate Targeting Membrane Immunoglobulin E-Positive Cells. International Journal of Molecular Sciences, 24(19); doi.org/10.3390/ijms241914997.
Sampath, V., Abrams, E.M., Adlou, B., Akdis, C., Akdis, M. & Brough, H.A. et al. (2021) Food allergy across the globe. The Journal of Allergy and Clinical Immunology, 148(6), 1347-1364; doi.org/10.1016/j.jaci.2021.10.018.
Scheerens, H., Putnam, W., Zheng, Y., Wang, Y., Mosesova, S. & Maciuca, R. et al. Treatment With MEMP1972A, An Anti-M1 Prime Monoclonal Antibody, Reduced Serum IgE In Healthy Volunteers And Patients With Allergic Rhinitis. In:, A6791-A6791; doi.org/10.1164/ajrccm-conference.2012.185.1_MeetingAbstracts.A6791.
Seifert, O., & Kontermann, R. E. (2022). GlycoTAIL and FlexiTAIL as Half-Life Extension Modules for Recombinant Antibody Fragments. Molecules, 27(10), 3272. doi.org/10.3390/molecules27103272
Sivori S, Meazza R, Quintarelli C, Carlomagno S, Della Chiesa M, Falco M, Moretta L, Locatelli F, Pende D, 2019, NK Cell-Based Immunotherapy for Hematological Malignancies, J Clin Med. 8(10):1702, doi: 10.3390/jcm8101702.
Tharp, M.D., Bernstein, J.A., Kavati, A., Ortiz, B., MacDonald, K. & Denhaerynck, K. et al. (2019) Benefits and Harms of Omalizumab Treatment in Adolescent and Adult Patients With Chronic Idiopathic (Spontaneous) Urticaria: A Meta-analysis of "Real-world" Evidence. JAMA Dermatology, 155(1), 29-38; doi.org/10.1001/jamadermatol.2018.3447.
Tom, R., Bisson, L. & Durocher, Y. (2008) Transfection of HEK293-EBNA1 Cells in Suspension with Linear PEI for Production of Recombinant Proteins. CSH Protocols, 2008(3), pdb.prot4977; doi.org/10.1101/pdb.prot4977.
Vafa O, Trinklein ND, 2020, Perspective: Designing T-Cell Engagers With Better Therapeutic Windows, Front. Oncol. 10: 446, doi: 10.3389/fonc.2020.00446
Vogel, F. (2022), Herstellung und Modifikation bispezifischer Antikörper gegen humanes Immunglobulin E. Dissertation, LMU Munich, Faculty of Medicine, doi:10.5282/edoc.30681 Wan, L., Chen, J.-B., Chen, H.H., Huang, J., Yu, H.-M. & Luo, S.-F. et al. (2010) Genetic variations in the C epsilon mX domain of human membrane-bound IgE. Immunogenetics, 62(5), 273-280; doi.org/10.1007/s00251-010-0437-0.
Voigt J, Meyer C, Bordusa F, (2022), Synthesis of Multiple Bispecific Antibody Formats with Only One Single Enzyme Based on Enhanced Trypsiligase, Int. J. Mol. Sci. 23, 3144, DOI: 10.3390/ijms23063144
Wang, J., Zhou, Y., Zhang, H., Hu, L., Liu, J. & Wang, L. et al. (2023) Pathogenesis of allergic diseases and implications for therapeutic interventions. Signal Transduction and Targeted Therapy, 8(1), 138;doi.org/10.1038/s41392-023-01344-4.
Ward, D.E., Fay, B.L., Adejuwon, A., Han, H. & Ma, Z. (2018) Chimeric Antigen Receptors Based on Low Affinity Mutants of FcεRI Re-direct T Cell Specificity to Cells Expressing Membrane IgE. Frontiers in Immunology, 9, 2231. Available from: https://doi.org/10.3389/fimmu.2018.02231
Warren, C.M., Aktas, O.N., Manalo, L.J., Bartell, T.R. & Gupta, R.S. (2023) The epidemiology of multifood allergy in the United States: A population-based study. Annals of Allergy, Asthma & Immunology : Official Publication of the American College of Allergy, Asthma, & Immunology, 130(5), 637-648.e5; doi.org/10.1016/j.anai.2022.12.031.
Wood, R.A., Togias, A., Sicherer, S.H., Shreffler, W.G., Kim, E.H. & Jones, S.M. et al. (2024) Omalizumab for the Treatment of Multiple Food Allergies. New England Journal of Medicine, 390(10), 889-899; doi.org/10.1056/NEJMoa2312382.
Wu, Z. & Cheung, N.V. (2018) T cell engaging bispecific antibody (T-BsAb): From technology to therapeutics. Pharmacology & Therapeutics, 182, 161-175; doi.org/10.1016/j.pharmthera.2017.08.005.
Wu, L.C. & Scheerens, H. (2014) Targeting IgE production in mice and humans. Current Opinion in Immunology, 31, 8-15. Available from: https://doi.org/10.1016/j.coi.2014.08.001.
Wu, L.C. & Zarrin, A.A. (2014) The production and regulation of IgE by the immune system. Nature Reviews. Immunology, 14(4), 247-259; doi.org/10.1038/nri3632.
Xiong, S., Jia, Y. & Liu, C. (2022) IgE-expressing long-lived plasma cells in persistent sensitization. Frontiers in Pediatrics, 10, 979012; doi.org/10.3389/fped.2022.979012.
Zhang, K., Saxon, A. & Max, E.E. (1992) Two unusual forms of human immunoglobulin E encoded by alternative RNA splicing of epsilon heavy chain membrane exons. The Journal of Experimental Medicine, 176(1), 233-243; doi.org/10.1084/jem.176.1.233.

## Claims

1. An antibody for use in a therapy of an IgE-associated disease, the antibody comprising
a) a first binding domain binding to the CεmX domain of the immunoglobulin E extracellular membrane-proximal domain, EMPD, the first binding domain comprising a complementarity-determining region, CDR, of the single-chain variable fragment antibody 2E3E10;
b) a second binding domain binding to an epitope on the surface of an immune effector cell.

2. The antibody for use in a therapy of an IgE-associated disease according to claim 1, wherein the first binding domain comprises at least two, at least three, at least four, at least five or six of the CDRs of the single-chain variable fragment antibody 2E3E10, more preferably at least one, two, three, four, five or all of the CDRs with amino acid sequences QSLLYSSNQKNY (SEQ ID NO: 2), WAS, QQYYSYPWT (SEQ ID NO:3), GYSITSGYY (SEQ ID NO: 4), ISYDGSN (SEQ ID NO: 5), ARDYGGFDY (SEQ ID NO: 6).

3. The antibody for use in a therapy of an IgE-associated disease according to claim 1 or 2, wherein the second binding domain is a binding domain binding to an epitope on CD3 or CD16.

4. The antibody for use in a therapy of an IgE-associated disease according to one of claims 1 to 3, having an amino acid sequence comprising at least 200 consecutive amino acids, preferably at least 210 consecutive amino acids, more preferably at least 220, 230 or 240 consecutive amino acids, particular preferred the full length of the sequence of scFv antibody 2E3E10 according to SEQ ID NO: 7, with the proviso that the antibody in any case comprises the CDRs of scFv antibody 2E3E10.

5. The antibody for use in a therapy of an IgE-associated disease according to one of the preceding claims, wherein the first binding domain comprising a complementarity-determining region, CDR, of the single-chain variable fragment antibody 2E3E10 is arranged further N-terminal than the second binding domain binding to an epitope on CD3 or CD16.

6. The antibody for use in a therapy of an IgE-associated disease according to one of the preceding claims, wherein the second binding domain
a) comprises a CDR of the blinatumomab anti-CD3 scFv antibody, preferably at least two, three, four, five, or six of the CDRs according to the amino acid sequences of SEQ ID NO 8-13, or a CDR of the single-domain anti-CD3 antibody CD3 3D-H11, preferably one, two or three of the CDRs according to the amino acid sequences of SEQ ID NO 15-17, or
b) comprises a CDR of the anti-CD16 antibody c21, preferably one, two or three of the CDRs according to the amino acid sequences of SEQ ID NO 19-21.

7. The antibody for use in a therapy of an IgE-associated disease according to claim 6, wherein the second binding domain has or comprises a) one of the amino acid sequences of SEQ ID NO: 14 or SEQ ID NO: 18 or b) the sequence of SEQ ID NO 22.

8. The antibody for use in a therapy of an IgE-associated disease according to one of the preceding claims, wherein the antibody comprises a third peptide domain conveying an extended plasma half-life compared to an antibody lacking the third peptide domain, and wherein the third peptide domain is preferably an anti-Human-Serum-Albumin-antibody, more preferably an anti-HSA-antibody having or comprising the amino acid sequence of SEQ ID NO: 31.

9. The antibody for use in a therapy of an IgE-associated disease according to one of the preceding claims, the antibody comprising a secretory signal peptide.

10. The antibody for use in a therapy of an IgE-associated disease according to one of the preceding claims, wherein the first and second binding domain are separated by a linker sequence, preferably by a 5-30 amino acids linker sequence, more preferably by a 5-20, 5-18, 5-15 or 5-12 amino acids linker sequence, more preferably by a 5-30, 5-20, 5-18, 5-15 or 5-12 amino acids GS linker or Whitlow/218 linker, more preferably by one of the linker sequences of SEQ ID NO: 24-28, 29-30.

11. The antibody for use in a therapy of an IgE-associated disease according to one of the preceding claims, having or comprising one of the amino acid sequences of SEQ ID NO: 33, 35, 37, 39 or 41.

12. A pharmaceutical composition or dosage form for use in a therapy of an IgE-associated disease, the pharmaceutical composition comprising an antibody according to one of claims 1 to 11, and a pharmaceutically acceptable carrier.

13. A nucleic acid encoding an antibody for use in a therapy of an IgE-associated disease according to one of claims 1 to 11.

14. The nucleic acid according to claim 13 having or comprising one of the sequences of SEQ ID NO: 32, 34, 36, 38, or 40.

15. The antibody of one of claims 1 to 11, or the pharmaceutical composition according to claim 12 or the nucleic acid of one of claims 13 or 14, wherein the therapy of an IgE-associated disease is an anti-allergy therapy.
